# EUROPEAN PATENT SPECIFICATION

(11) **EP 1 712 642 B1**
(45) Date of publication and mention of the grant of the patent: **02.06.2010**
(21) Application number: 05710143.8
(22) Date of filing: 04.02.2005
(51) Int. Cl.: C12Q 1/68, C12Q 1/02, G01N 33/48, G01N 33/53, A61K 31/365, A61K 31/496, A61P 35/00, C07D 407/06

(54) **METHOD OF EXAMINING SENSITIVITY OF CANCER CELL TO ANTICANCER AGENT**
VERFAHREN ZUR UNTERSUCHUNG DER EMPFINDLICHKEIT EINER KREBSZELLE GEGENÜBER EINEM ANTIKREBSMITTEL
PROCEDE D EXAMEN DE LA SENSIBILITE D UNE CELLULE CANCER EUSE A UN AGENT ANTICANCEREUX

(30) Priority: 06.02.2004 JP 2004030106; 11.03.2004 JP 2004069384
(43) Date of publication of application: 18.10.2006
(73) Proprietor: Eisai R&D Management Co., Ltd., Tokyo 112-8088 (JP); MERCIAN CORPORATION, Chuo-ku, Tokyo 104-8305 (JP)
(72) Inventor: UENAKA, Toshimitsu, Eisai R&D Management Co.,Ltd., Tsukuba-shi, Ibaraki (JP); IWATA, Masao, Eisai R&D Management Co.,Ltd., Tsububa-shi, Ibaraki (JP)
(74) Representative: HOFFMANN EITLE
(86) International application number: PCT/JP2005/002091
(87) International publication number: WO 2005/075681

(56) References cited:
- WO-A1-02/42493
- WO-A1-02/060890
- WO-A1-03/099813
- WO-A1-2004/011661
- KANETA YASUYUKI ET AL: "Prediction of sensitivity to STI571 among chronic myeloid leukemia patients by genome-wide cDNA microarray analysis" JAPANESE JOURNAL OF CANCER RESEARCH, AMSTERDAM, NL, vol. 93, no. 8, August 2002 (2002-08), pages 849-856, XP002260979
- SEKI-ASANO M ET AL: "ISOLATION AND CHARACTERIZATION OF A NEW 12-MEMBERED MACROLIDE FD-895" JOURNAL OF ANTIBIOTICS, JAPAN ANTIBIOTICS RESEARCH ASSOCIATION, TOKYO, JP, vol. 47, no. 12, December 1994 (1994-12), pages 1395-1401, XP002951021 ISSN: 0021-8820
- UENAKA T. ET AL: 'E7107: a novel planienolide D derivative III. Study for predictive factors.' THE JAPANESE CANCER ASSOCIATION SOKAI KIJI. 25 August 2004, page 499, XP002992116

## Description

### Technical Field

The present invention relates to an assay method for predicting sensitivity of a cancer cell to a compound useful as an anticancer drug represented by the formula (I) (hereinafter referred to as the present compound).

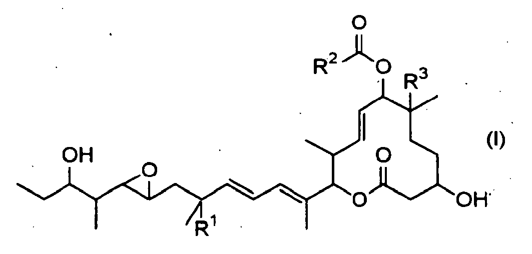

### Background Art

Cancer chemotherapy has been conventionally conducted with anticancer drugs, which have been proven to be effective on each kind of organ cancers, alone or in combination with other drugs of them. Additionally, in specific cancer types, standard therapies have been established through large-scale randomized controlled trials which are becoming popular in recent years. However, these therapies have achieved their response rates just in general 20-50%. Although More than half of patients cannot have therapeutic effects, chemotherapy associated with the risk of adverse reactions is used in the present situation (JACR Monograph No. 7, 10-19, 2002).

For attempt to sort patients depending on effectiveness of anticancer drugs, methods "test for drug sensitivity" have been conventionally tried. The tests for anticancer drug sensitivity include SDI test (Jpn J Cancer Res. 85: 762-765, 1994), CD-DST (Jpn J Cancer Res. 92: 203-210, 2001), HDRA (Clin Cancer Res. 1: 305-311, 1995) or the like, and all of these methods have common procedures that a cancer cell is sampled and exposed to an anticancer drug to evaluate sensitivity to the anticancer drug. However, these methods have problems that the cancer cells should be kept alive in a test tube, test results and clinical effects are not necessarily in concord, or the like, and so their execution rate remains at approximately 0.5% (summary math of questionnaire by Japan Research Society for Appropriate Cancer Chemotherapy, 1996).

In relation to molecular-targeted drugs which target cancer cell-specific molecules, a case that sensitivity to a drug is successfully predicted by molecular diagnosis such as the Herceptin test has been reported (Clin Cancer Res. 7: 1669-1675, 2001). However, in relation to other drugs, sensitivity can not be predicted by single target molecule alone, suggesting that a plurality of molecules often specify sensitivity (Nat Genet. 24: 236-244, 2000). Recently developed cDNA microarray technology allowed expression analysis of genes in super-large amount and therefore comprehensive analysis of sensitivity-specifying factors. For example, therapeutic effects of Gleevec, a drug for treating chronic myelogenous leukemia can be predicted by expression analysis of the 15 to 30 gene clusters, which has been proposed from the result of cDNA microarray analysis (Jpn J Cancer Res. 93: 849-56, 2002).

The future development of an anticancer drug used in cancer chemotherapy should aim to provide a combination with a diagnostic procedure for evaluating sensitivity to the drug before treatment, and thus it is important to find out a molecule for molecular diagnosis which allows prediction of sensitivity to the drug to be developed.

### Disclosure of the Invention

Cytocidal activities of macrolide FD-895, which is a derivative of the present compound, against various cultured cancer cell lines including adriamycin resistant HL-60, have been reported in the Journal of Antibiotics, Japan Antibiotics Research Association, Tokyo, JP, vol. 47, 1994, 1395-1401.

The present inventor found, in WO 02/060890, WO 03/099813, and WO 04/011661, that the present compound expressed extremely potent antitumor activity to human breast cancer cell line (BSY-1), and also to human colon cancer cell line (WiDr).

On the other hand, the present compound was found out to be less effective to small cell lung cancer cell line (Calu-1) compared to the above cancer cell strains, demonstrating that antitumor activity of the present compound depended on cancer cells.

If molecules are proven to be associated with the sensitivity of cancer cells to the present compound, and cancer cells sampled from a cancer patient by biopsy or the like can be examined for the expression of these molecules, then the present compound can be selectively administered only to the cancer patients who are expected to benefit from antitumor activity of the present compound and so the enhanced therapeutic effect is expected to reduce unnecessary adverse effects.

In researching molecular characteristics of BSY-1 strain which has high sensitivity to the present compound, the present inventors focused on molecules which control G1/S transition in the cell cycle which is proposed to be disrupted in breast and lung cancers. Four molecules including p16 and pRB which are tumor-suppressor genes, and cyclin D1 and cyclin E which compete with or negatively control them were examined with respect to their respective protein expression levels. As a result, BSY-1 showed the molecular characteristics of reduced expression of pRB, positive expression of p16 and high level expression of cyclin E.

25 human cancer cell lines including BSY-1, WiDr and Calu-1 were examined to clarify their respective characteristics with respect to reduced expression or mutation of pRB (hereinafter referred to as low level expression of pRB), positive expression of p16 or high level expression of cyclin E. Meanwhile, mice implanted with these cancer cells were used to weigh the antitumor activity of the present compound on them. Many of the human cancer cell lines which had at least one of these characteristics exhibited high sensitivity to the present compound, whereas most of the cancer cell lines lacking in these characteristics were less sensitive.

Mouse implanted with seven strains with characteristics of low level expression of pRB and high level expression of cyclin E and those implanted with six strains with characteristics of positive expression of p16 and high level expression of cyclin E were administrated with the present compound. As a result, four strains of them especially showed complete elimination of tumors, and one strain showed a long-term sustained reduction of tumors, though did not a complete elimination.

It has been found out that a cancer cell can be examined its characteristics with respect to low level expression of pRB, positive expression of p16 or high level expression of cyclin E to predict the sensitivity of the cancer cell to the present compound. The finding completed the present invention.

Namely, the present invention relates to the followings.
1. An assay method to predict sensitivity of a cancer cell to a compound represented by the following formula I, comprising using any one index of:
   1) expression of pRB is reduced;
   2) p16 is expressed;
   3) expression of cyclin E is enhanced;
   4) expression of pRB is reduced and expression of cyclin E is enhanced; or
   5) p16 is expressed and expression of cyclin E is enhanced: Formula (I) 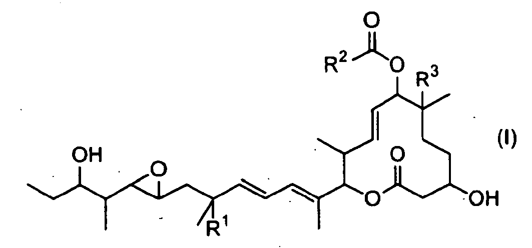
      (Wherein, R¹ represents
         (1) hydrogen atom or
         (2) a hydroxyl group;
      R³ represents
         (1) hydrogen atom,
         (2) a hydroxyl group or
         (3) a C₁₋₆ alkoxy group; and
      R² represents
         (1) hydrogen atom,
         (2) a C₁₋₆ alkyl group which may have a substituent,
         (3) a C₇₋₁₀ aralkyl group which may have a substituent,
         (4) a 5-membered to 14-membered heteroaralkyl group which may have a substituent,
         (5) the formula (II): 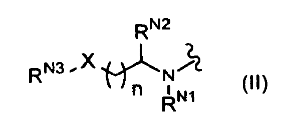 (wherein,
            A)
               n represent an integer of 0 to 4;
               X represents
                  i) -CHR^{N4}-,
                  ii) -NR^{N5}- or
                  iii) -O-;
               R^{N1} and R^{N2} are the same as or different from each other and each represents
                  i) hydrogen atom or
                  ii) a C₁₋₆ alkyl group;
               R^{N3} and R^{N4} are the same as or different from each other and each represents
                  i) hydrogen atom,
                  ii) a C₁₋₆ alkyl group which may have a substituent,
                  iii) an unsaturated C₂₋₁₀ alkyl group which may have a substituent,
                  iv) a C₁₋₆ alkoxy group which may have a substituent,
                  v) a C₆₋₁₄ aryl group which may have a substituent,
                  vi) a 5-membered to 14-membered heteroaryl group which may have a substituent,
                  vii) a C₇₋₁₀ aralkyl group which may have a substituent,
                  viii) a C₃₋₈ cycloalkyl group which may have a substituent,
                  ix) a C₄₋₉ cycloalkylalkyl group which may have a substituent,
                  x) a 5-membered to 14-membered heteroaralkyl group which may have a substituent,
                  xi) a 5-membered to 14-membered non-aromatic heterocyclic group which may have a substituent,
                  xii) -NR^{N6}R^{N7} (wherein, R^{N6} and R^{N7} are the same as or different from each other and each represents hydrogen atom or a C₁₋₆ alkyl group) or
                  xiii) R^{N3} and R^{N4} are bound together with the carbon atom to which they are bound to form a 5-membered to 14-membered non-aromatic heterocyclic group which may have a substituent (the non-aromatic heterocyclic group may have a substituent);
               R^{N5} represents
                  i) hydrogen atom,
                  ii) a C₁₋₆ alkyl group which may have a substituent,
                  iii) an unsaturated C₂₋₁₀ alkyl group which may have a substituent,
                  iv) a C₆₋₁₄ aryl group which may have a substituent,
                  v) a 5-membered to 14-membered heteroaryl group which may have a substituent,
                  vi) a C₇₋₁₀ aralkyl group which may have a substituent,
                  vii) a C₃₋₈ cycloalkyl group which may have a substituent,
                  viii) a C₄₋₉ cycloalkylalkyl group which may have a substituent,
                  ix) a 5-membered to 14-membered heteroaralkyl group which may have a substituent,
                  x) a 5-membered to 14-membered non-aromatic heterocyclic group which may have a substituent, or
                  xi) R^{N3} and R^{N5} are bound together with the nitrogen atom to which they are bound to form a 5-membered to 14-membered non-aromatic heterocyclic group which may have a substituent (the non-aromatic heterocyclic group may have a substituent),
            B)
               X, n, R^{N3}, R^{N4} and R^{N5} represent the above defined groups; and R^{N1} and R^{N2} represent a 5-membered to 14-membered non-aromatic heterocyclic group which R^{N1} and R^{N2} are bound together to form and which may have a substituent,
            C)
               X, n, R^{N2}, R^{N4} and R^{N5} represent the above defined groups, and R^{N1} and R^{N3} represent a 5-membered to 14-membered non-aromatic heterocyclic group which R^{N1} and R^{N3} are bound together to form and which may have a substituent, or
            D)
               X, n, R^{N1}, R^{N4} and R^{N5} represent the above defined groups; and R^{N2} and R^{N3} represent 5-membered to 14-membered non-aromatic heterocyclic group which R^{N2} and R^{N3} are bound together to form and which may have a substituent), or
         (6) the formula (III): 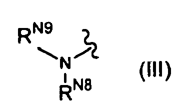 (wherein, R^{N8} and R^{N9} are the same as or different from each other and each represents
            i) hydrogen atom,
            ii) a C₁₋₆ alkyl group which may have a substituent,
            iii) a C₆₋₁₄ aryl group which may have a substituent,
            iv) a 5-membered to 14-membered heteroaryl group which may have a substituent
            v) a C₇₋₁₀ aralkyl group which may have a substituent, or
            vi) a 5-membered to 14-membered heteroaralkyl group which may have a substituent)).
2. The assay method according to the item 1, wherein R² is
   1) hydrogen atom;
   2) a C₁₋₆ alkyl group which may have a substituent,
   3) a C₇₋₁₀ aralkyl group which may have a substituent or
   4) a 5-membered to 14-membered heteroaralkyl group which may have a substituent.
3. The assay method according to the item 1, wherein R² is represented by the following formula (IV): 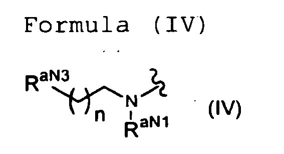 (wherein n represents an integer of 0 to 4;
   R^{aN1} represents
      1) hydrogen atom or
      2) a C₁₋₆ alkyl group; and
   R^{aN3} represents
      1) hydrogen atom
      2) a N-C₁₋₆ alkylamino group,
      3) an N,N-di-C₁₋₆ alkylamino group,
      4) ethylmethylamino group,
      5) pyridyl group,
      6) pyrrolidin-1-yl group,
      7) piperidin-1-yl group,
      8) morpholin-4-yl group or
      9) 4-methylpiperazin-1-yl group).
4. The assay method according to the item 1, wherein R² is represented by the following formula (V): 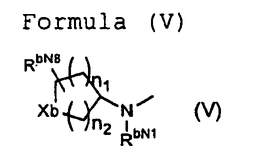 (wherein n₁ and n₂ are the same as or different from each other and each represents an integer of 0 to 4;
   X_{b} represents
      1) -CHR^{bN4}-,
      2) -NR^{bN5}- or
      3) -O-;
   R^{bN1} represents
      1) hydrogen atom or
      2) a C₁₋₆ alkyl group;
   R^{bN8} represents
      1) hydrogen atom,
      2) a C₁₋₆ alkyl group,
      3) a C₆₋₁₄ aryl group or
      4) a C₇₋₁₀ aralkyl group;
   R^{bN4} represents
      1) hydrogen atom,
      2) a C₁₋₆ alkyl group which may have a substituent,
      3) an unsaturated C₂₋₁₀ alkyl group which may have a substituent,
      4) a C₁₋₆ alkoxy group which may have a substituent,
      5) a C₆₋₁₄ aryl group which may have a substituent,
      6) a 5-membered to 14-membered heteroaryl group which may have a substituent,
      7) a C₇₋₁₀ aralkyl group which may have a substituent,
      8) a C₃₋₈ cycloalkyl group which may have a substituent,
      9) a C₄₋₉ cycloalkylalkyl group which may have a substituent,
      10) a 5-membered to 14-membered heteroaralkyl group which may have a substituent,
      11) NR^{bN6}R^{bN7} (wherein R^{bN6} and R^{bN7} are the same as or different from each other and each represents hydrogen atom or a C₁₋₆ alkyl group) or
      12) a 5-membered to 14-membered non-aromatic heterocyclic group which may have a substituent; and
   R^{bN5} is
      1) hydrogen atom,
      2) a C₁₋₆ alkyl group which may have a substituent,
      3) an unsaturated C₂₋₁₀ alkyl group which may have a substituent,
      4) a C₆₋₁₄ aryl group which may have a substituent,
      5) a 5-membered to 14-membered heteroaryl group which may have a substituent
      6) a C₇₋₁₀ aralkyl group which may have a substituent,
      7) a C₃₋₈ cycloalkyl group which may have a substituent,
      8) a C₄₋₉ cycloalkylalkyl group which may have a substituent,
      9) a 5-membered to 14-membered heteroaralkyl group which may have a substituent or
      10) a 5-membered to 14-membered non-aromatic heterocyclic group which may have a substituent).
5. The assay method according to the item 1, wherein R² is represented by the following formula (VI): 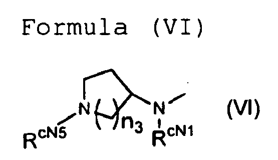 (wherein n₃ represents an integer of 1 or 2;
   R^{cN1} represents
      1) hydrogen atom or
      2) a C₁₋₆ alkyl group; and
   R^{cN5} represents
      1) hydrogen atom or
      2) a C₁₋₆ alkyl group).
6. The assay method according to the item 1, wherein R² is represented by the following formula (VII): 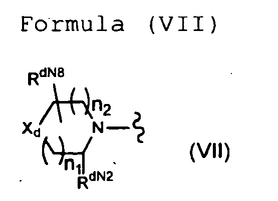 (wherein n₁ and n₂ are the same as or different from each other and each represents an integer of 0 to 4;
   X_{d} represents
      1) -CHR^{dN4}-,
      2) -NR^{dN5}- or
      3) -O-; and
   R^{dN2} represents
      1) hydrogen atom or
      2) a C₁₋₆ alkyl group;
   R^{aN8} represents
      1) hydrogen atom,
      2) a C₁₋₆ alkyl group,
      3) a C₆₋₁₄ aryl group or
      4) a C₇₋₁₀ aralkyl group;
   R^{dN4} represents
      1) hydrogen atom,
      2) a C₁₋₆ alkyl group which may have a substituent,
      3) an unsaturated C₂₋₁₀ alkyl group which may have a substituent,
      4) a C₁₋₆ alkoxy group which may have a substituent,
      5) a C₆₋₁₄ aryl group which may have a substituent,
      6) a 5-membered to 14-membered heteroaryl group which may have a substituent,
      7) a C₇₋₁₀ aralkyl group which may have a substituent,
      8) a C₃₋₈ cycloalkyl group which may have a substituent,
      9) a C₄₋₉ cycloalkylalkyl group which may have a substituent,
      10) a 5-membered to 14-membered heteroaralkyl group which may have a substituent,
      11) -NR^{dN6}R^{dN7} (wherein R^{dN6} and R^{dN7} are the same as or different from each other and each represents hydrogen atom or a C₁₋₆ alkyl group) or
      12) a 5-membered to 14-membered non-aromatic heterocyclic group which may have a substituent; and
   R^{dN5} represents
      1) hydrogen atom,
      2) a C₁₋₆ alkyl group which may have a substituent,
      3) an unsaturated C₂₋₁₀ alkyl group which may have a substituent,
      4) a C₆₋₁₄ aryl group which may have a substituent,
      5) a 5 to 14-membered ring heteroaryl group which may have a substituent,
      6) a C₇₋₁₀ aralkyl group which may have a substituent,
      7) a C₃₋₈ cycloalkyl group which may have a substituent,
      8) a C₄₋₉ cycloalkylalkyl group which may have a substituent,
      9) a 5-membered to 14-membered heteroaralkyl group which may have a substituent or
      10) a 5-membered to 14-membered non-aromatic heterocyclic group which may have a substituent).
7. The assay method according to the item 1, wherein R² is represented by the following formula (VIII): 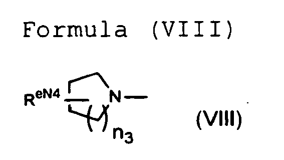 (wherein n₃ represents an integer of 1 to 3; and
   R^{eN4} represents
      1) amino group,
      2) a N-C₁₋₆ alkylamino group,
      3) pyrrolidin-1-yl group,
      4) piperidin-1-yl group or
      5) morpholin-4-yl group).
8. The assay method according to the item 1, wherein R² is represented by the following formula (IX): 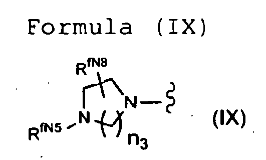 (wherein n₃ represents an integer of 1 to 3;
   R^{fN8} represents
      1) hydrogen atom,
      2) a C₁₋₆ alkyl group,
      3) a C₆₋₁₄ aryl group or
      4) a C₇₋₁₀ aralkyl group; and
   R^{fN5} is
      1) hydrogen atom,
      2) a C₁₋₆ alkyl group which may have a substituent,
      3) a C₃₋₈ cycloalkyl group which may have a substituent,
      4) a 3-membered to 8-membered non-aromatic heterocyclic group which may have a substituent,
      5) a C₆₋₁₄ aryl group which may have a substituent,
      6) a 5-membered to 14-membered heteroaryl group which may have a substituent,
      7) a C₇₋₁₀ aralkyl group which may have a substituent,
      8) a 5-membered to 14-membered heteroaralkyl group which may have a substituent or
      9) a C₄₋₉ cycloalkylalkyl group which may have a substituent).
9. The assay method according to the item 1, wherein R² is represented by the following formula (X): 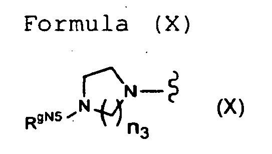 (wherein n₃ represents an integer of 1 to 3; and
   R^{gN5} represents
      1) hydrogen atom
      2) a C₁₋₆ alkyl group which may be substituted,
      3) a C₃₋₈ cycloalkyl group which may be substituted,
      4) a C₄₋₉ cycloalkylalkyl group which may be substituted,
      5) a C₇₋₁₀ aralkyl group which may be substituted,
      6) a pyridyl group which may be substituted or
      7) a tetrahydropyranyl group which may be substituted).
10. The assay method according to the item 1, wherein the compound represented by the formula (I) is any one compound selected from the group consisting of the following compounds:
   1) (8E,12E,14E)-7-acetoxy-3,6,21-trihydroxy-6,10,12,16,20-pentamethyl-18,19-epoxytricosane-8,12,14-trien-11-olide,
   2) (8E,12E,14E)-7-((4-cycloheptylpiperazin-1-yl)-carbonyl)oxy-3,6,16,21-tetrahydroxy-6,10,12,16,20-pentamethyl-18,19-epoxytricosane-8,12,14-trien-11-olide,
   3) (8E,12E,14E)-3,6,16,21-tetrahydroxy-7-((4-isopropylpiperazin-1-yl)carbonyl)oxy-6,10,12,16,20-pentamethyl-18,19-epoxytricosane-8,12,14-trien-11-olide and
   4) (8E,12E,14E)-3,6,16,21-tetrahydroxy-6,10,12,16,20-pentamethyl-7-((4-methylpiperazin-1-yl)carbonyl)oxy-18,19-epoxytricosane-8,12,14-trien-11-olide
11. The assay method according to the item 1, comprising assaying a reduced expression of pRB, an expression of p16 or an enhanced expression of cyclin E by measuring the levels of their respective encoding mRNAs.
12. The assay method according to the item 11, wherein the method for measuring the level of the mRNAs is a quantitative RT-PCR method.
13. The assay method according to the item 11, wherein the method for measuring the level of the mRNAs is a DNA tip method.
14. The assay method according to the item 1, comprising assaying a reduced expression of pRB, an expression of p16 or an enhanced expression of cyclin E by measuring the levels of their respective proteins.
15. The assay method according to the item 14, wherein the method for measuring the levels of their respective proteins is a western blot method.
16. The assay method according to the item 14, wherein the method for measuring the levels of their respective proteins is an immunohistostaining method.
17. The assay method according to the item 14, wherein the method for measuring the levels of their respective proteins is an ELISA method.
18. A kit for use in the assay method according to the item 12, comprising a primer that contains at least 15 consecutive base sequences of the pBR, p16 or cyclin E genes.
19. A kit for use in the assay method according to the item 15, 16 or 17, comprising an antibody to the pRB, p16 or cyclin E.

A cancer cell can be examined to clarify its characteristics with respect to low level expression of pRB, positive expression of p16 or high level expression of cyclin E allows prediction of sensitivity of the cancer cell to the present compound, allowing selective administration of the present compound only to cancer patients who are expected to benefit from antitumor activity of the present compound. The enhanced therapeutic effect and the reduced unnecessary adverse effect are expected.

### Best Modes for Carrying Out the Invention

In the present description, the pRB is a protein with a molecular weight of approximately 110 KDa which is encoded by a base sequence described in Genbank Accession No. NM000321. The RB gene encoding pRB was isolated as a tumor-suppressor gene which mutates in retinoblastoma (Proc. Natl Acad. Sci. USA 84: 9059-9063, 1987), and then its mutation has been confirmed in various malignant cells.

The p16 is a protein with a molecular weight of approximately 16 KDa which is coded by a base sequence described in Genbank Accession No. HM000077. The p16 binds to CDK4 and CDK6, and suppresses CDK4/6 from phosphorylating pRB, thereby to help pRB in keeping the activity (Science 264: 436-440, 1994).

The cyclin E is a protein with a molecular weight of approximately 52 KDa which is coded by a base sequence described in Genbank Accession No. M74093, and is increased in expression level temporarily at the G1/S transition of the cell cycle to play a leading role for cell-cycle progression. However, canceration is reported to increase its absolute level, and to disrupt a pattern of its temporarily increase of the expression (Int. J Cancer 104: 369-75, 2003).

In addition, a reverse correlation between p16 and pBR in expression level has been reported in many cancer types, and, therefore, the positive expression of p16 and the low level expression of pRB are considered to be attributed to the same event (EMBO J. 14: 503-511, 1995).

The present compound can be manufactured by methods described in WO 02/060890, WO 03/099813 and WO 04/011661. Furthermore, for the present compound to have anticancer activity is described in WO 02/060890, WO 03/099813 and WO 04/011661.

The present invention is an assay method to predict for sensitivity of a cancer cell to the present compound, comprising examining characteristics of the cancer cell with respect to low level expression of pRB, positive expression of p16 or high level expression of cyclin E. The cancer cell may be a cell sampled from the cancer tissue or a cell cultured in vitro, and is preferably a cell collected from the cancer tissue by biopsy, and more preferably the cancer tissue is a cancer tissue collected by biopsy for cancer diagnosis.

A cancer patient, whose cancer cell collected has been judged to be highly sensitive to the present compound by the method of the present invention, can be selected to administrate with the present compound in order to expect the patient to benefit by the anticancer activity of the present compound. Consequently, the enhanced therapeutic effects and reduced unnecessary adverse effects are expected.

In the present invention, the method for examining the characteristics of the cancer cell with respect to low level expression of pBR, positive expression of p16 or high level expression of cyclin E may be a method for measuring the level of transcribed mRNAs or a method for measuring the level of translated proteins. The method for measuring the level of mRNA includes a RT-PCR method and a DNA tip method, and the method for measuring the level of protein includes a western blot method, an immunohistostaining method, and an ELISA method. Any method for measuring an expression level can be used, and the present invention is not limited to these examples.

Also, the present invention includes an embodiment wherein mutation of the protein (Reports concerning mutation of pRB: Proc Natl Acad Sci USA 87: 6922-6, 1990; Proc Natl Acad Sci USA 87: 6-10, 1990) which is substantially equal to reduction of the expression is detected as the mutated DNA/mRNA by analyzing the base sequences or electrophoresis mobility of the DNA/mRNA (Oncogene 8: 1913-9, 1993), or an embodiment wherein the mutated protein is detected by an antibody or electrophoresis mobility which can identify mutation.

Hereinafter, the method of measuring the expression level of pRB, p16, and cyclin E from the sampled tumor tissues will be described in detail, and the expression level can be also measured from the cultured cells by the same method, and so the present invention is not limited to these methods.

### 1. RT-PCR method:

An mRNA is prepared from a sampled tumor tissue by a routine procedure (Experimental Medicine, extra number, "PCR and its application" 8: 1063-1071, 1990). The expression level of pRB/p16/cyclin E can be measured using the prepared mRNA as a template by RT-PCR. In RT-PCR, reverse transcription reaction and subsequent PCR (RT-PCR) can be carried out using Real Time RT-PCR Core Kit (Takara Code PR032A) or the like.

For measuring the expression level by quantitation of mRNA, quantitative RT-PCR is preferable. Although there have been reports of various methods of quantitative RT-PCR (Genome Res. 6: 986-994, 1996), competitive RT-PCR is desirable, wherein RT-PCR is conducted in coexistence with a known amount of competitive template RNA which is amplified preferably by the same primer, and the amounts of the both amplified products are determined to compare.

A primer for detecting an mRNA coding pRB/p16/cyclin E (called target mRNA) is prepared on the basis of the sequence information described in the above-mentioned Genbank Accession Number. And a competitive template RNA which has the sequence complementary to the primer but has a different molecular weight or restriction enzyme cleavage site from those of the target mRNA for determination is prepared. The primer is targeted for a site where mutation frequently occurs (the site in pRB was reported in Proc Natl Acad Sci USA 87: 6922-6, 1990; Proc Natl Acad Sci USA 87: 6-10, 1990), thereby only the mutated protein or only the normal protein can be determined.

RT-PCR is conducted by adding the diluted known amount of competitive template RNA to the mRNA sample prepared from the tumor tissue and adding the primer thereto. Based on the molecular weights of the amplified products or a restriction enzyme-digested product thereof, it is determined whether it is derived from the target mRNA or the competitive template RNA, and a quantitative value of the target mRNA is calculated by a ratio of both the amplified products and an amount of the added competitive template RNA. The mRNA level may be measured as a relative value in comparison with an actin mRNA or 18S rRNA or the like which are universally expressed. The mRNA level of pRB/p16/cyclin E in highly and lowly sensitive cancer cells is measured to decide a cutoff value, and then compared with the cutoff value to judge the level of expression.

In addition, appearance of bands with different mobility on electrophoresis from that of the standard cell or detection of mutated proteins is decisive in low level expression.

The cancer cell, which has characteristics of low level expression of pRB, positive expression of p16 or high level expression of cyclin E, preferably in the case of low level expression of pRB and high level expression of cyclin E, or positive expression of p16 and high level expression of cyclin E, is judged to be highly sensitive to the present compound.

### 2. DNA tip method:

An mRNA is extracted from a sampled tumor tissue by a routine procedure (Experimental Medicine, extra number, "PCR and its application" 8: 1063-1071, 1990), labeled with fluorescence by reverse transcription reaction, and the synthesized labeled cDNA is hybridized on a microarray (IntelliGene Human Cancer CHIP Ver. 4.0, X102, TAKARA BIO INC.) to which the oligonucleotide of a cancer-associated gene is spotted (Nature genetics supplement 21; 10-14; 1999) .

The tumor tissue sample and the normal tissue sample are labeled with different fluorescent dyes such as Cy3 (red) and Cy5 (green) to judge in which sample of them genes increase. Computer analysis software makes image display possible. For example, it shows a red display when the gene hybridized with the labeled cDNA is amplified more from the tumor tissue sample than from the normal cell sample, a yellow display when equally amplified, and a green display when less amplified. Intensity of these signals is digitized by the computer analysis software, thereby the data of the gene expression ratio between them (Cy3=cancer tissue/Cy5=normal tissue) are calculated, and if the expression ratio is 2 or more or 1/2 or less, preferably 3 or more or 1/3 or less, a difference in expression is judged to be significant.

When the pRB is green, the p16 is yellow or red, or the cyclin E is red, the cancer cell is judged to be highly sensitive to the present compound. Preferably, when the pRB is green and the cyclin E is red, or the p16 is yellow or red and the cyclin E is red, the cancer cell is judged to be highly sensitive to the present compound.

### 3. Western blot method

The anti-pRB antibody/anti-p16 antibody/anti-cyclin E antibody used in the following western blot method, immunohistostaining method and ELISA method may be commercially available antibodies. For example, for the anti-pRB antibody, Rb Antibody can be obtained from Cell Signaling Technology, Inc. (Catalog No. 9302), for the anti-p16 antibody, p16 (C-20) can be obtained from Santa Cruz Biotechnology, Inc. (Catalog No. sc-468), and for the anti-cyclin E antibody, Purified anti-human Cyclin E can be obtained from Pharmingen/BD Company (Catalog No. 554182).

Also, the antibodies can be prepared by immunization with an antigen prepared based on information described in the above-mentioned Genbank Accession No. The antigen can be obtained by preparing a pRB/p16/cyclin E expression vector based on sequence information described in the above-mentioned Genbank Accession Number and purifying the pRB/p16/cyclin E protein from the expression cell to which the vector is introduced. These proteins are preferably expressed as fusion proteins to facilitate purification. And as the other method, the antigen can be prepared by synthesizing a peptide based on sequence information described in the above-mentioned Genbank Accession Number and combining it with a carrier protein. The antibody can be purified from a serum which is obtained from an animal immunized with the antigen, or a culture of a hybridoma which is prepared from the obtained antibody-producing cell.

Additionally, for detecting mutation of pRB/p16/cyclin E, above all pRB, a mutated protein with mutation described in Proc Natl Acad Sci USA 87: 6922-6, 1990; Proc Natl Acad Sci USA 87 : 6-10, 1990 is expressed, or a peptide with mutation sites is synthesized and an animal is immunized with the protein/peptide, thereby to obtain an antibody which specifically responds to the mutated protein. On the contrary, an antibody which responds to normal proteins but not to mutated proteins can be also prepared by immunization with the normal protein. The method of preparing the antibody is described in Methods in Enzymology 182, p663-679.

A sample for SDS-PAGE including the pRB/p16/cyclin E is prepared from the sampled tumor tissue. Specifically, the tumor tissue is homogenized using a cell preparing solution (preferably containing each protease inhibitor and 10% of glycerol), mixed with an equal amount of sample buffer for SDS-PAGE, and heat-treated at 97°C for five minutes, thereby to obtain the sample for SDS-PAGE, for example. The western blot method can be conducted according to the method described in Methods in Enzymology 182, p679-688, nevertheless, can be concretely conducted as below, for example.

A certain amount of each sample is separated by SDS-PAGE, transferred to Hybond ECL membrane, reacted with the anti pRB/p16/cyclin E antibody, and reacted with the enzyme-labeled second antibody, to detect the pRB/cyclin E/p16 transferred to the Hybond ECL membrane by enzyme activity. As preferable controls, the standard cell which shows expression of pRB/p16/cyclin E, more preferably MDA-MB435 cell which shows expression of all of them is applied at the same time, and comparison with expression in MDA-MB435 cell determines expressivity.

In addition, appearance of bands with different mobility on electrophoresis from that of the standard cell or detection of mutated proteins is decisive in low level expression.

The cancer cell, which has characteristics of low level expression of pRB, positive expression of p16 or high level expression of cyclin E, preferably in the case of low level expression of pRB and high level expression of cyclin E, or positive expression of p16 and high level expression of cyclin E, is judged to be highly sensitive to the present compound.

### 4. Immunohistostaining method

Immunohistostaining method can be conducted according to a method described in Experimental Medicine, separate volume (Immunostaining in post-genome era, in situ hybridization, 1997), and can be concretely conducted as below, for example.

The sampled tumor tissue is infiltrated with an embedding agent to make a block, which is then sliced into 2-8 µm thick pieces, and applied on a slide to prepare a tissue specimen. The anti-pRB antibody, anti-p16 antibody, or anti-cyclin E antibody is reacted with the tissue specimen and then stained by avidin-biotin interaction in immunoenzymatic technique (J. Histochem. Cytochem. 27; 1131-1139, 1979). Low level expression of pRB, positive expression of p16 and high level expression of cyclin E is judged by comparing staining intensity of pRB/p16/cyclin E between the tumor tissue and the normal tissue in the stained tissue specimen. Detection of mutated proteins is decisive in low level expression.

The cancer cell, which has characteristics of low level expression of pRB, positive expression of p16 or high level expression of cyclin E, preferably in the case of low level expression of pRB and high level expression of cyclin E, or positive expression of p16 and high level expression of cyclin E, is judged to be highly sensitive to the present compound.

### 5. ELISA method

ELISA method can be conducted according to a method described in Antibodies A Laboratory Manual (Ed Harlow et al, Cold Spring Harbor Laboratory), and can be concretely conducted as below, for example.

A fraction including the pRB/p16/cyclin E is prepared from the sampled tumor tissue. Concretely, the tumor tissue is homogenized with a cell preparing solution (preferably containing each protease inhibitor and 10% of glycerol), to which a 1% of NP-40 is added to obtain a soluble fraction and a residue. The soluble fraction is directly used for the ELISA method, or the residue is subjected to extraction using 0.5M NaCl and used for the ELISA method after the NaCl concentration is lowered to 0.1-0.15 M.

The soluble fraction and the NaCl-extracted fraction are applied to a 96-well plate coated with the anti-pRB antibody, anti-p16 antibody, or anti-cyclin E antibody to react with the antibody. The pRB, p16 or cyclin E binding to the coated antibody is sandwiched by an enzyme-labeled antibody to each protein, and the pRB, p16 or cyclin E expressed in the tumor tissue is determined by measuring activity of the binding enzyme.

The amounts of the pRB/p16/cyclin E in highly sensitive and lowly sensitive cancer cells are measured to decide a cutoff value, and comparison with the cutoff level judges expressivity.

The cancer cell, which has characteristics of low level expression of pRB, positive expression of p16 or high level expression of cyclin E, preferably in the case of low level expression of pRB and high level expression of cyclin E, or positive expression of p16 and high level expression of cyclin E, is judged to be highly sensitive to the present compound.

### Brief Description of Drawings

Fig. 1 is an analysis result of the expression levels of pRB/cyclin E/p16/cyclin D1 in each human cancer cell line, by western blotting.
Fig. 2 is an analysis result of the expression levels of pRB/p16/cyclin E in the strains of SY-1, H-526 and H-460 by immunohistostaining.
Fig. 3 is an analysis result of the expression levels of p16/cyclin E in a clinical specimen of endometrial cancer by immunohistostaining.
Fig. 4 is a graph showing correlations between the p16 mRNA level and T/C%, and between the p16 mRNA level and the expression level of the p16 protein.

### Examples

Hereinafter, examples for demonstrating usefulness of the present invention will be described, but are exemplary, and the invention is not limited to the following concrete examples in any case.

### Example 1 Expression analysis of cell cycle-related molecules in 25 human cancer cell lines including BSY-1 and WiDr

In 25 human cancer cell lines including BSY-1 which showed high sensitivity to (8E,12E,14E)-7-acetoxy-3,6,21-trihydroxy-6, 10, 12, 16, 20-pentamethyl-18, 19-epoxytricosane-8,12,14-trien-11-olide (hereinafter referred to as compound 1) represented by the following formula II in WO 02/060890, and WiDr which showed sensitivity to the compounds which is represented by the formula I in WO 03/099813 wherein R1 is a hydroxyl group, expression of the cell cycle-related molecules, pRB, p16, cyclin E and cyclin D1 was analyzed by the western blot method.

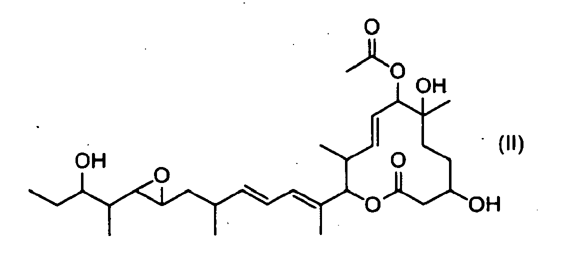

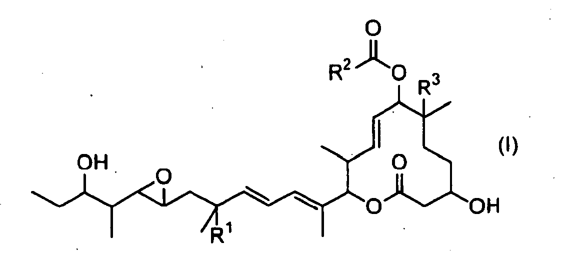

The 25 human cancer cell lines were subcutaneously implanted to the bodysides of nude mice, the tumors were collected at the time of a tumor volume of 100 mm³ or larger, and homogenized with a cell preparing solution including each protease inhibitor (Leupeptin, p-APMSF, EDTA, o-NaV04) and 10% of glycerol, when the cell preparing solution per weight of the tumor sample was adjusted to have a constant amount. Each cell preparing solution is mixed with an equal amount of sample buffer for SDS-PAGE, and heat-treated at 97°C for five minutes, to obtain the sample for SDS-PAGE. A certain amount of each sample for SDS-PAGE is separated by SDS-PAGE, transferred to Hybond ECL membrane, reacted with the anti pRB/cyclin E/p16/cyclin D1 antibody. Then, it was reacted with the HRP-labeled second antibody, and mixed with a chemiluminescence substrate (Super Signal: PIERCE), to detect a band of the pRB/cyclin E/p16/cyclin D1 using Image Master VDS-CL (Amersham Pharmacia).

As a control for relative comparison of the expression level of pRB/p16/cyclin E/ cyclin D1 in each caner cell line, cells of MDA-MB435 which showed expression of all molecules were applied to all SDS-PAGEs, and detection was performed in the same manner.

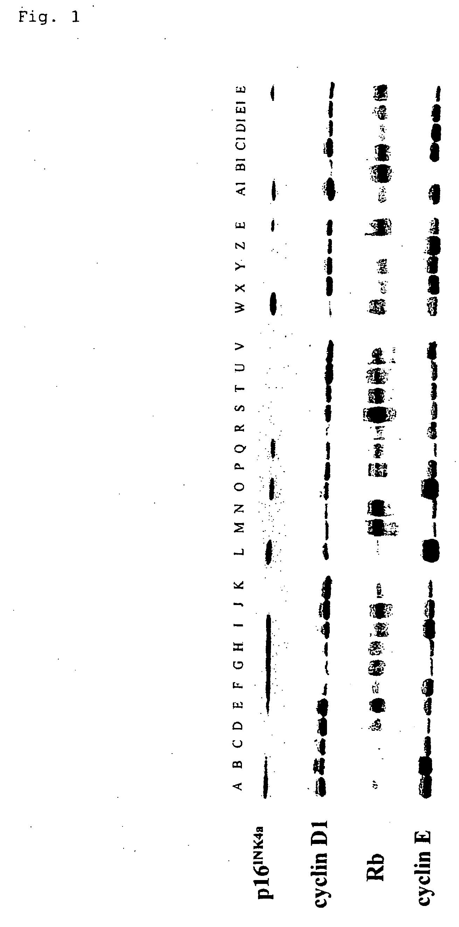

The results are shown in Fig. 1. BSY-1 which showed high sensitivity had characteristics of deletion of pRB, expression of p16 and high level expression of cyclin E, therefore, whether or not these characteristics were associated with sensitivity to the present compound *in vivo* was studied.

In Fig. 1, codes, A-Z, E, A1-E1 and E show their respective human cancer cell lines as follows.

| | | |
|---|---|---|
| **A : BSY-1 ( breast )** | **K : FaDu ( head & neck )** | **U : DLD-1 ( colorectal )** |
| **B : MDA-MB-468 ( breast )** | **L : LC-6-JCK ( NSCLC )** | **V : PC-3 ( prostate )** |
| **C : HBC-4 ( breast)** | **M : Lu99 ( NSCLC )** | **W : NCl-H510A ( SCLC )** |
| **D : KPL-4 ( breast )** | **N : NC-H460 ( NSCLC )** | **X : NCl-H522 ( SCLC )** |
| **E : MDA-MB-435 ( breast )** | **O : NCl-H596 ( NSCLC )** | **Y : WiDr ( colorectal )** |
| **F : NCl-H146 ( SCLC )** | **P : Calu-1 ( NSCLC )** | **Z : DU145 ( prostate )** |
| **G : NCI-H69 ( SCLC )** | **Q : PC14 ( NSCLC )** | **A1: DMS114 ( NSCLC )** |
| **H : NCl-H526 ( SCLC )** | **R : HT-29 ( colorectal )** | **B1: Colo320DM( colorectal )** |
| **I : NIH :OVCAR-3 ( ovarian )** | **S : SW620 ( colorectal )** | **C1: COL-3 ( colorectal )** |
| **J : SK-OV**-**3 ( ovarian )** | **T : KM12 ( colorectal )** | **D1: Lovo ( colorectal )** |
| | | **E1: MKN45 ( stomach )** |

### Example 2 Correlation between expression of pRB/p16/cyclin E/cyclin D1 and sensitivity to the present compound

Regarding the 25 human cancer cell lines which were studied on expression of pRB/p16/cyclin E/cyclin D1 in Example 1, sensitivity to the representative of the present compounds [(8E,12E,14E)-7-((4-cycloheptylpiperazin-1-yl)carbonyl)oxy-3,6,16,21-tetrahydroxy-6,10,12,16,20-pentamethyl-18,19-epoxytricosane-8,12,14-trien-11-olide] (referred to as Compound 2) represented by the following formula (III) was evaluated.

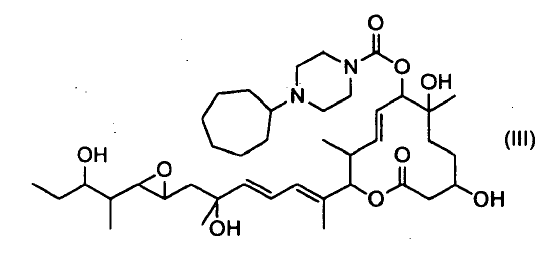

The 25 human cancer cells proliferated in culture flasks or in the subcutaneous tissues of the nude mice were subcutaneously implanted to the bodysides of nude mice, and the mice were classified into a group of 5 control mice and a group of 5 treated mice of the compound 2 so that the average tumor volume of each group was uniform at the time of a tumor volume of 100 mm³ or larger. The mice of the treated group were intravenously infused at 10 mg/kg/day for five days, and the mice of the control group were not treated or infused with the vehicle. The tumor volume was measured from day 1, subsequently measured over time on Day 5, 8, 12, 15, and a relative tumor volume ratio (T/C%)was estimated.

Correlations between expression of pRB/p16/cyclin E/cyclin D1 and sensitivity to the compound 2 are shown in Table 1. The expression level of pRB/cyclin E/p16/cyclin D1 in the human cancer cell line analyzed in Example 1 was relatively evaluated, where no expression was represented by "-", expression was represented by "+ to +++" according to the intensity, and shown together with the results of the tumor volume ratio (T/C%). Furthermore, the expression level of pRB in small cell lung cancer cell line, ovarian cancer line and prostate cancer cell line (Oncogene 9, 3375-3378, 1994; Prostate 21, 145-52, 1992; Exp Cell Res, 233, 233-9, 1997) which had been reported to express mutated pRB, were described with "()".

**Table 1 Expression level of pRB/p16/cyclin E/cyclin D1 and sensitivity to the compound 2**

| Cell line | pRB | p16 | cyclin E | cyclin D1 | T/C% |
|---|---|---|---|---|---|
| BSY-1 | - | ++ | +++ | +++ | 0 |
| MDA-MB468 | - | + | +++ | ++ | 0 |
| LC-6-JCK | - | ++ | +++ | + | 0 |
| OVCAR3 | (+) | ++ | +++ | ++ | 0 |
| NCI-H146 | (±) | ++ | +++ | ± | 1 |
| NCI-H69 | (++) | ++ | ± | ± | 1 |
| NCI-H526 | (+) | ++ | + | ± | 1 |
| PC-3 | ++ | - | ++ | +++ | 2 |
| FaDu | + | - | ± | +++ | 3 |
| WiDr | + | - | +++ | +++ | 4 |
| HBC4 | - | - | ++ | ++ | 5 |
| Lu99 | +++ | - | ± | ± | 8 |
| NCI-H510 | (+) | ++ | + | ± | 10 |
| NCI-H596 | - | ++ | +++ | + | 18 |
| KPL-4 | + | - | + | +++ | 23 |
| SK-OV-3 | +++ | - | ++ | +++ | 27 |
| DU145 | - | - | +++ | ++ | 28 |
| MDA-MB435 | +++ | + | ++ | +++ | 28 |
| HT-29 | + | - | + | ± | 28 |
| SW620 | +++ | - | + | ++ | 28 |
| NCI-H460 | +++ | - | + | ± | 33 |
| KM12 | ++ | - | + | ++ | 34 |
| NCI-H522 | + | - | +++ | +++ | 42 |
| DLD-1 | ++ | - | + | +++ | 47 |
| Calu-1 | + | - | ++ | ++ | 55 |

Of the 25 examined human cancer cell lines, 11 cancer cell lines in which bands could not detected by western blotting (represented by "-") or mutation or deletion had been reported to have in literature information (represented with parentheses), were classified into a group of low level expression of pRB including 7 strains (64%) which showed T/C%≤1%. On the other side, 14 strains of pRB expression did not include the cancer cell lines of T/C%≤1% (p=0.0006), demonstrating that sensitivity to the compound 2 could be assayed by low level expression of pRB.

In addition, 7 strains (70%) of 10 cancer cells of positive expression of p16 showed T/C%≤1%, whereas, 15 strains of no expression of p16 did not include the cancer cell line of T/C%≤1% (p=0.0002), and so sensitivity to the compound 2 could be assayed by positive expression of p16, like low level expression of pRB.

Of the 25 examined cancer cell lines, 11 strains were classified into a group of low level expression of pRB (the bands could not detected by western blotting, or mutation and deletion had been reported in literature information) including 9 strains which expressed p16. On the contrary, 9 of 10 strains of p16 expression showed low level expression of pRB, and the cancer cells of positive expression of p16 were approximately coincided with the cancer cells of low level expression of pRB, as reported (EMBO J. 14: 503-511, 1995).

In terms of high level expression of cyclin E, 5 (56%) of 9 strains of high level expression (+++) showed T/C%≤1%, whereas, 2 (13%) of 16 strains of the cancer cells of no high level expression showed T/C%≤1%, consequently the cancer cells of high level expression of cyclin E were highly sensitive (p=0.02).

The effects of the compound 2 on 5 strains including 4 strains which showed complete elimination of the tumor (T/C%=0%) and NCI-H146 which was not eliminated but showed consecutive reduction of the tumor for a long term, were considered to be curative, and all of these strains showed the characteristics of low level expression of pRB, expression of p16 and high level expression of cyclin E.

Thus correlation between the two characteristics of low level expression of pRB and high level expression of cyclin E and the curative effects of the compound 2 indicated that the curative effects appeared in 5 strains (71%) of 7 cancer cell lines with both characteristics but did not appear in the other 18 cancer cell lines (p=0.0001). Furthermore, in the cancer cells with two characteristics of positive expression of p16 and high level expression of cyclin E, the curative effects appeared in 5 strains (83%) of 6 cancer cell lines with both characteristics, but did not appear in the other 19 cancer cell lines (p=0.00002). High level expression of cyclin E became a more useful indicator by being combined with the indicators of low level expression of pRB or positive expression of p16.

From these results, sensitivity of the cancer cell lines to the compound 2 proved predictable by examination of the characteristics of low level expression of pRB, expression of p16 and high level expression of cyclin E.

### Example 3 Assay for sensitivity of the cancer cell line to three representative compounds

Regarding the compound 1 which showed high antitumor activity to BSY-1 in WO 02/060890, and the representative compounds of the present compounds represented by the formula 1 which showed antitumor activity to WiDr in WO 03/099813, (8E,12E,14E)-3,6,16,21-tetrahydroxy-7-((4-isopropylpiperazin-1-yl)carbonyl) oxy-6,10,12,16,20-pentamethyl-18,19-epoxytricosane-8,12,14-trien-11-olide (the following formula IV, hereinafter referred to as the compound 3) and (8E,12E,14E)-3,6,16,21-tetrahydroxy-6,10,12,16,20-pentamethyl-7-((4-methylpiperazin-1-yl)-carbonyl)oxy-18,19-epoxytricosane-8,12,14-trien-11-olide (the following formula V, hereinafter referred to as the compound 4), correlation between the characteristics of low level expression of pRB, expression of p16 and high level expression of cyclin E and sensitivity was evaluated in the human cancer cell line.

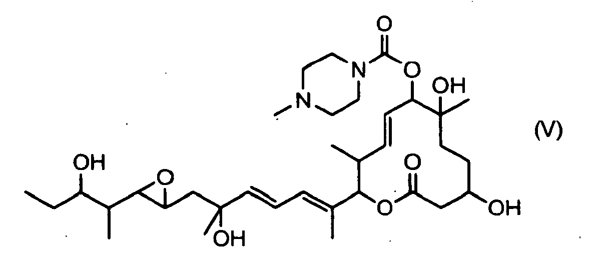

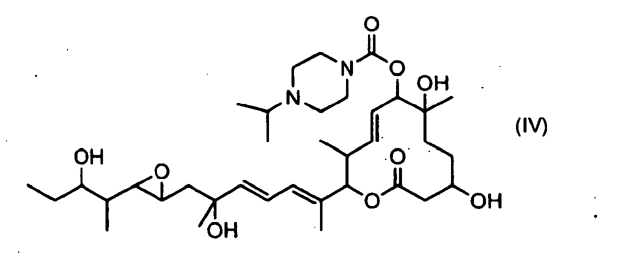

The cancer cells proliferated in culture flasks or in the subcutaneous tissues of the nude mice were subcutaneously implanted to the bodysides of nude mice, and the mice were classified into a group of 5 control mice and a group of 5 treated mice of the compounds so that the average tumor volume of each group was uniform at the time of a tumor volume of 100 mm³ or larger. The mice of the treated group were intravenously infused at 10 mg/kg/day for five days, and the mice of the control group were not treated or infused with the vehicle. The tumor volume was measured from Day 1, and subsequently measured over time on Day 5, 8, 12, 15, and the relative tumor volume ratio was estimated. However, the experiment was basically discontinued at Day 15 in evaluation of the compound 1. In Table 2, the T/C% of the compounds 1, 2, 4 and the compound 2 in Example 2 to each cancer cell are shown.

As shown in Table 2, although the compound 1 showed somewhat high T/C%, all tested compounds showed antitumor activities, indicating that sensitivity of each human cancer cell line to these compounds denoted the same tendency. When correlation between T/C% of the compounds 1, 3 and 4 and that of the compound 2 was estimated, proportions as high as 0.724, 0.948, 0.923 were obtained. This suggested that each cancer cell line had common sensitivity among compounds represented by the formula I.

**Table 2 Correlation between anti-tumor effect of the compound and those of the compounds 1, 3 and 4**

| Cell line | Tumor volume ratio (T/C%) | | | |
|---|---|---|---|---|
| | Compound 2 | Compound 1 | Compound 3 | Compound 4 |
| BSY-1 | 0 | 0 | 2 | N.D. |
| LC-6-JCK | 0 | N.D. | 0 | N.D. |
| OVCAR3 | 0 | 23 | N.D. | N.D. |
| PC-3 | 2 | 20 | 4 | 3 |
| FaDu | 3 | 33 | 1 | 5 |
| WiDr | 4 | 29 | 4 | 7 |
| NCI-H596 | 18 | 76 | N.D. | N.D. |
| DU145 | 28 | 26 | 34 | 26 |
| MDA-MB435 | 28 | 50 | N.D. | N.D. |
| HT-29 | 28 | 58 | 26 | 46 |
| SW620 | 28 | N.D. | 19 | N.D. |
| NCI-H460 | 33 | 53 | N.D. | N.D. |
| NCI-H522 | 42 | 64 | N.D. | N.D. |
| DLD-1 | 47 | 59 | N.D. | N.D. |

### Example 4 Analysis of expression of pRB/p16/cyclin E in BSY-1, H-526, H-460 by immunohistostaining

Expression of the cell cycle-related molecules pRB/p16/cyclin E was analyzed by the immunohistostaining method in BSY-1 which showed high sensitivity until remission, H526 which showed high sensitivity without remission and H460 which showed low sensitivity, to the compound 2.

The above mentioned 3 human cancer cell lines were subcutaneously implanted to the bodysides of nude mice, the tumors were collected at the time of a tumor volume of 100 mm³ or larger, and fixed with a neutralized 10% formalin for one day to prepare a paraffin-embedded block. The paraffin block was sliced into 4 µm thick pieces and applied on a slide, to prepare a specimen for immunohistostaining. The specimen for immunohistostaining was deparaffinized and heated in an incubator to activate antigenicity, blocked an endogenous peroxidase, and then reactd with a first antibody to the pRB/p16/cyclin E at a room temperature for one hour. The specimen was reacted with a peroxidase-labeled anti-mouse or -rabbit second antibody and microscopically observed by visualizing the activity of the peroxidase using DAB as a colorimetric substrate.

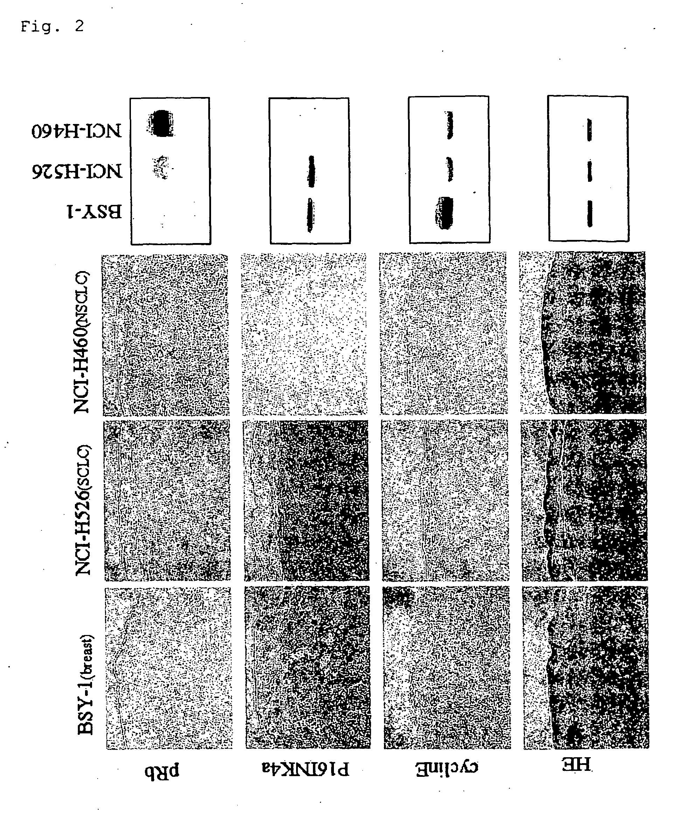

The results are shown in Fig. 2. The same result as that in the western blot method was clearly obtained in immunohistostaining. The result indicated that expression of the cell cycle-related molecules, pRB/p16/cyclin E could be analyzed even by using the paraffin-embedded block specimen commonly used in clinical practice.

### Example 5 Analysis of expression of pRB/p16/cyclin E in clinical specimens by immunohistostaining

Expression of the cell cycle-related molecules p16/cyclin E was analyzed by the immunohistostaining method using paraffin-embedded clinical specimens (12 normal tissues and 12 cancer tissues) purchased from SuperBioChips Laboratories Inc.

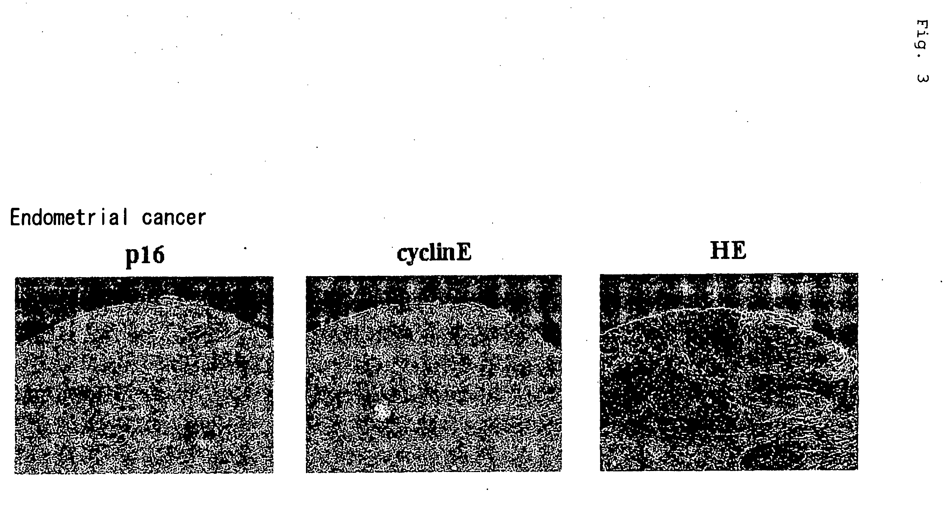

The paraffin-embedded clinical specimens were subjected to immunohitostaining using the antibodies against the p16/cyclin E in the same manner as Example 4. As a result, in a part of the cancer tissues, overexpression of the p16 (3 of 12 cases) and the cyclin E (1 of 12 cases) was observed, although not observed in the normal tissues. In Fig. 3, the result of staining in endometrial cancer which both p16 and cyclin E were overexpressed was shown. The result indicated that the cancer tissues which the cell cycle-related molecules, p16/cyclin E were overexpressed could be selected even by using the paraffin-embedded block specimen in clinical practice.

### Example 6 Immunohistostaining for expression of p16/cyclin E in the clinical specimens (2)

In addition to Example 5, in many more cancers (10 samples each cancer; gastric cancer, esophagus cancer, lung cancer, colon cancer, thyroid cancer, renal cancer, breast cancer, hepatic cancer, bladder cancer, ovarian cancer, pancreatic cancer, prostate cancer, endometrial cancer, gallbladder cancer, laryngeal cancer, cervical carcinoma, malignant lymphoma and malignant melanoma; total of 180 samples), expression of the cell-cycle-related molecules p16/cyclin E was analyzed by the immunohistostaining method using paraffin-embedded clinical tumor specimens purchased from SuperBioChips Laboratories Inc., in the same way as Examples 4 and 5.

The paraffin-embedded clinical specimens were subjected to immunohistostaining using the antibodies against the p16/cyclin E in the same manner as Examples 4 and 5, and the characteristic "overexpression of the p16 and cyclin E" was found in all of 10 cases of cervical carcinoma, in 4 of 10 cases of ovarian cancer, and in 3 of 10 cases of breast cancer as shown in Table 2, indicating that the cancer tissues of "overexpression of the p16 and cyclin E" could be selected using the paraffin-embedded block specimen in clinical practice and that certain cancer tissues could frequently have the characteristic of "overexpression of the p16 and cyclin E".

**Table 3 Frequency of appearance of the "overexpression of the p16 and cyclin E" in each tumor specimens**

| cancers | Frequency of the overexpression | cancers | Frequency of the overexpression |
|---|---|---|---|
| gastric cancer | 1/10 | ovarian cancer | 4/10 |
| esophagus cancer | 1/10 | pancreatic cancer | 1/10 |
| lung cancer | 0/10 | prostate cancer | 0/8* |
| colon cancer | 1/10 | endometrial cancer | 1/10 |
| thyroid cancer | 0/10 | gallbladder cancer | 1/10 |
| renal cancer | 0/9* | laryngeal cancer | 1/10 |
| breast cancer | 3/10 | cervical carcinoma | 10/10 |
| hepatic cancer | 0/10 | malignant lymphoma | 0/10 |
| bladder cancer | 2/9* | malignant melanoma | 2/9* |

| | | | |
|---|---|---|---|
| * : Since the samples being unable to evaluate (such as those not including the cancer cells or those peeled off from the slide) were excluded, the evaluated cases were less than 10. Example 7 Detection of overexpression of the p16 by RT-PCR | | | |

The 28 human cancer cell lines were subcutaneously implanted to the bodysides of nude mice, the tumors were collected at the time of a tumor volume of 100 mm³ or larger, and 3 tumors each cancer were pooled. The tumors were frozen with liquid nitrogen, and total RNAs were extracted using TRIzol (SIGMA) and purified with RNeasy mini Kit (OIAGEN). After cDNA was synthesized with Taqman^{(R)} reverse transcription reagents (ABI), the p16 mRNA level in each tumor was measured by Sequence detection systems (7900HT, ABI) using Taqman^{(R)} gene expression assays (ABI) of p16 as a probe and Taqman^{(R)} Gold RT-PCR reagents as a reaction reagent. The obtained value was corrected by the value of 18S rRNA to estimate the relative levels of the p16 mRNA.

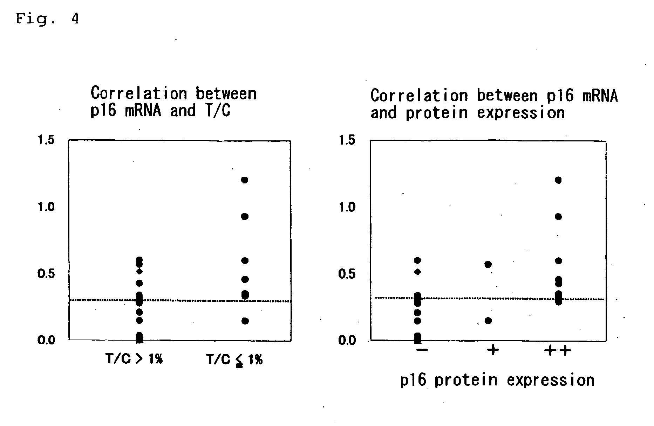

The relative levels of the p16 mRNAs in the human cancer cell lines were compiled from the expression level of the p16 protein determined by the western blot method in Example 1 and the antitumor action T/C% determined in Example 2, and shown in Table 3. Furthermore, Fig. 4 is a graph depicting correlations between the p16 mRNA levels and T/C%, and between the p16 mRNA levels and the expression level of p16 protein.

As shown in Table 3 and Fig. 4, of the 12 strains of high level expression with the ≥0.3 of the relative level of p16 mRNA, the 6 strains (50%) showed T/C%=1%, whereas of the 13 strains of <0.3, just one strain showed T/C%≤1% (p=0.02). Measurement of the p16 mRNA level demonstrated that sensitivity to the compound 2 could be assayed by screening cancers of "overexpression of p16".

Additionally, Table 3 and Fig. 4 showed correlation between the expression level of p16 protein, that is, the 8 strains which showed the p16 mRNA level and overexpression of p16 protein (++), had 0.5786±0.3259 of the relative mRNA level of p16, while on the other hand, the 2 strains which showed expression of p16 protein (+) had 0.3610, and the 15 strains which showed no expression of p16 protein had 0.2073±0.2424. The methods of detecting the p16 mRNA like RT-PCR are considered to give the same results as those by the method of detecting expression of p16 protein.

**Table 4 Relation between the p16 mRNA levels and the p16 protein expression levels**

| Cell line | relative levels of p16 mRNA | p16 protein expression | T/C% |
|---|---|---|---|
| BSY-1 | 1.2065 | + + | 0 |
| MDA-MB468 | 0.1503 | + | 0 |
| LC-6-JCK | 0.6046 | + + | 0 |
| OVCAR-3 | 0.9336 | + + | 0 |
| NCl-H146 | 0.4642 | + + | 1 |
| NCl-H69 | 0.3556 | + + | 1 |
| NCl-H526 | 0.3372 | + + | 1 |
| PC-3 | 0.5174 | - | 2 |
| FaDu | 0.0060 | - | 3 |
| WiDr | 0.2110 | - | 4 |
| HBC4 | 0.0377 | - | 5 |
| Lu99 | 0.0000 | - | 8 |
| NCl-H510 | 0.4302 | + + | 10 |
| NCl-H596 | 0.2971 | + + | 18 |
| KPL-4 | 0.2822 | - | 23 |
| SK-OV-3 | 0.0000 | - | 27 |
| DU145 | 0.6043 | - | 28 |
| MDA-MB435 | 0.5716 | + | 28 |
| HT-29 | 0.2137 | - | 28 |
| SW620 | 0.3177 | - | 28 |
| NCH-H460 | 0.0001 | - | 33 |
| KM12 | 0.3392 | - | 34 |
| NCl-H522 | 0.0001 | - | 42 |
| DLD-1 | 0.0000 | - | 47 |
| Calu-1 | 0.1499 | - | 55 |

### Industrial Applicability

Characteristics of a cancer cell with respect to low level expression of pRB, positive expression of p16 or high level expression of cyclin E can be examined to evaluate sensitivity of the cancer cell to the present compound.

Thus, the present compound can be selectively used in the preparation of a medicament for the selective treatment of cancer in a cancer patient who is expected to benefit from the antitumor activity of the present compound, so that enhanced therapeutic effects and reduced unnecessary adverse effects may be expected.

## Claims

1. An assay method to predict sensitivity of a cancer cell to a compound represented by the following formula I, comprising using any one index of:
1) expression of pRB is reduced;
2) p16 is expressed;
3) expression of cyclin E is enhanced;
4) expression of pRB is reduced and expression of cyclin E is enhanced; or
5) p16 is expressed and expression of cyclin E is enhanced: 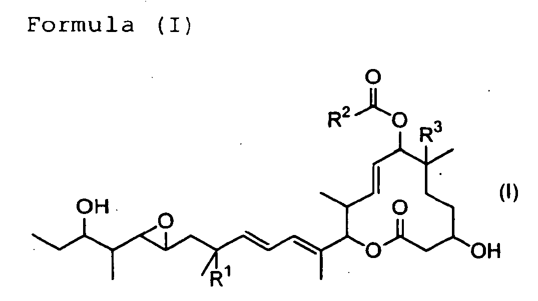
(Wherein, R¹ represents
(1) hydrogen atom or
(2) a hydroxyl group;
R³ represents
(1) hydrogen atom,
(2) a hydroxyl group or
(3) a C₁₋₆ alkoxy group; and
R² represents
(1) hydrogen atom,
(2) a C₁₋₆ alkyl group which may have a substituent,
(3) a C₇₋₁₀ aralkyl group which may have a substituent,
(4) a 5-membered to 14-membered heteroaralkyl group which may have a substituent,
(5) the formula (II): 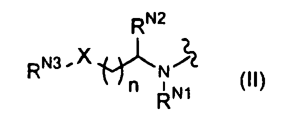 (wherein,
A)
n represent an integer of 0 to 4;
X represents
i) -CHR^{N4}-,
ii) -NR^{N5}- or
iii) -O-;
R^{N1} and R^{N2} are the same as or different from each other and each represents
i) hydrogen atom or
ii) a C₁₋₆ alkyl group;
R^{N3} and R^{N4} are the same as or different from each other and each represents
i) hydrogen atom,
ii) a C₁₋₆ alkyl group which may have a substituent,
iii) an unsaturated C₂₋₁₀ alkyl group which may have a substituent,
iv) a C₁₋₆ alkoxy group which may have a substituent,
v) a C₆₋₁₄ aryl group which may have a substituent,
vi) a 5-membered to 14-membered heteroaryl group which may have a substituent,
vii) a C₇₋₁₀ aralkyl group which may have a substituent,
viii) a C₃₋₈ cycloalkyl group which may have a substituent,
ix) a C₄₋₉ cycloalkylalkyl group which may have a substituent,
x) a 5-membered to 14-membered heteroaralkyl group which may have a substituent,
xi) a 5-membered to 14-membered non-aromatic heterocyclic group which may have a substituent,
xii) -NR^{N6}R^{N7} (wherein, R^{N6} and R^{N7} are the same as or different from each other and each represents hydrogen atom or a C₁₋₆ alkyl group) or
xiii) R^{N3} and R^{N4} are bound together with the carbon atom to which they are bound to form a 5-membered to 14-membered non-aromatic heterocyclic group which may have a substituent (the non-aromatic heterocyclic group may have a substituent);
R^{N5} represents
i) hydrogen atom,
ii) a C₁₋₆ alkyl group which may have a substituent,
iii) an unsaturated C₂₋₁₀ alkyl group which may have a substituent,
iv) a C₆₋₁₄ aryl group which may have a substituent,
v) a 5-membered to 14-membered heteroaryl group which may have a substituent,
vi) a C₇₋₁₀ aralkyl group which may have a substituent,
vii) a C₃₋₈ cycloalkyl group which may have a substituent,
viii) a C₄₋₉ cycloalkylalkyl group which may have a substituent,
ix) a 5-membered to 14-membered heteroaralkyl group which may have a substituent,
x) a 5-membered to 14-membered non-aromatic heterocyclic group which may have a substituent, or
xi) R^{N3} and R^{N5} are bound together with the nitrogen atom to which they are bound to form a 5-membered to 14-membered non-aromatic heterocyclic group which may have a substituent (the non-aromatic heterocyclic group may have a substituent),
B)
X, n, R^{N3}, R^{N4} and R^{N5} represent the above defined groups; and R^{N1} and R^{N2} represent a 5-membered to 14-membered non-aromatic heterocyclic group which R^{N1} and R^{N2} are bound together to form and which may have a substituent,
C)
X, n, R^{N2}, R^{N4} and R^{N5} represent the above defined groups, and R^{N1} and R^{N3} represent a 5-membered to 14-membered non-aromatic heterocyclic group which R^{N1} and R^{N3} are bound together to form and which may have a substituent, or D)
X, n, R^{N1}, R^{N4} and R^{N5} represent the above defined groups; and R^{N2} and R^{N3} represent 5-membered to 14-membered non-aromatic heterocyclic group which R^{N2} and R^{N3} are bound together to form and which may have a substituent), or
(6) the formula (III): 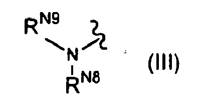 (wherein, R^{N8} and R^{N9} are the same as or different from each other and each represents
i) hydrogen atom,
ii) a C₁₋₆ alkyl group which may have a substituent,
iii) a C₆₋₁₄ aryl group which may have a substituent,
iv) a 5-membered to 14-membered heteroaryl group which may have a substituent
v) a C₇₋₁₀ aralkyl group which may have a substituent, or
vi) a 5-membered to 14-membered heteroaralkyl group which may have a substituent)).

2. The assay method according to claim 1, wherein R² is
1) hydrogen atom;
2) a C₁₋₆ alkyl group which may have a substituent,
3) a C₇₋₁₀ aralkyl group which may have a substituent or
4) a 5-membered to 14-membered heteroaralkyl group which may have a substituent.

3. The assay method according to claim 1, wherein R² is represented by the following formula (IV): 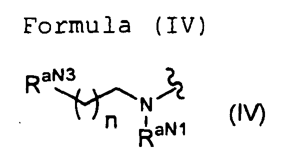 (wherein n represents an integer of 0 to 4;
R^{aN1} represents
(1) hydrogen atom or
(2) a C₁₋₆ alkyl group;
R^{aN3} represents
(1) hydrogen atom
(2) a N-C₁₋₆ alkylamino group,
(3) a N,N-di-C₁₋₆ alkylamino group,
(4) ethylmethylamino group,
(5) pyridyl group,
(6) pyrrolidin-1-yl group,
(7) piperidin-1-yl group,
(8) morpholin-4-yl group or
(9) 4-methylpiperazin-1-yl group).

4. The assay method according to claim 1, wherein R² is represented by the following formula (V): 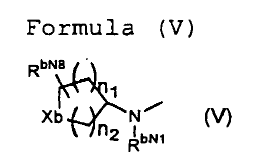 (wherein n₁ and n₂ are the same as or different from each other and each represents an integer of 0 to 4;
X_{b} represents
1) -CHR^{bN4}-,
2) -NR^{bN5}- or
3) -O-;
R^{bN1} represents
1) hydrogen atom or
2) a C₁₋₆ alkyl group;
R^{bN8} represents
1) hydrogen atom,
2) a C₁₋₆ alkyl group,
3) a C₆₋₁₄ aryl group or
4) a C₇₋₁₀ aralkyl group;
R^{bN4} represents
1) hydrogen atom,
2) a C₁₋₆ alkyl group which may have a substituent,
3) an unsaturated C₂₋₁₀ alkyl group which may have a substituent,
4) a C₁₋₆ alkoxy group which may have a substituent,
5) a C₆₋₁₄ aryl group which may have a substituent,
6) a 5-membered to 14-membered heteroaryl group which may have a substituent,
7) a C₇₋₁₀ aralkyl group which may have a substituent,
8) a C₃₋₈ cycloalkyl group which may have a substituent,
9) a C₄₋₉ cycloalkylalkyl group which may have a substituent,
10) a 5-membered to 14-membered heteroaralkyl group which may have a substituent,
11) -NR^{bN6}R^{bN7} (wherein R^{bN6} and R^{bN7} are the same as or different from each other and each represents hydrogen atom or a C₁₋₆ alkyl group) or
12) a 5-membered to 14-membered non-aromatic heterocyclic group which may have a substituent; and
R^{bN5} is
1) hydrogen atom,
2) a C₁₋₆ alkyl group which may have a substituent,
3) an unsaturated C₂₋₁₀ alkyl group which may have a substituent,
4) a C₆₋₁₄ aryl group which may have a substituent,
5) a 5-membered to 14-membered heteroaryl group which may have a substituent
6) a C₇₋₁₀ aralkyl group which may have a substituent,
7) a C₃₋₈ cycloalkyl group which may have a substituent,
8) a C₄₋₉ cycloalkylalkyl group which may have a substituent,
9) a 5-membered to 14-membered heteroaralkyl group which may have a substituent or
10) a 5-membered to 14-membered non-aromatic heterocyclic group which may have a substituent).

5. The assay method according to claim 1, wherein R² is represented by the following formula (VI): 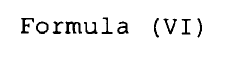 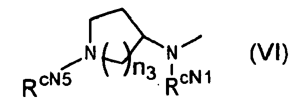 (wherein n₃ represents an integer of 1 or 2;
R^{CN1} represents
(1) hydrogen atom or
(2) a C₁₋₆ alkyl group;
R^{CN5} represents
(1) hydrogen atom or
(2) a C₁₋₆ alkyl group).

6. The assay method according to claim 1, wherein R² is represented by the following formula (VII): 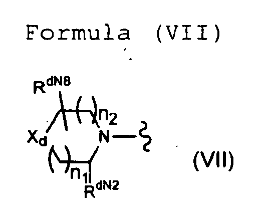 (wherein n₁ and n₂ are the same as or different from each other and each represents an integer of 0 to 4;
X_{d} represents
1) -CHR^{dN4}-,
2) -NR^{dN5}- or
3) -O-; and
R^{dN2} represents
1) hydrogen atom or
2) a C₁₋₆ alkyl group;
R^{dN8} represents
1) hydrogen atom,
2) a C₁₋₆ alkyl group,
3) a C₆₋₁₄ aryl group or
4) a C₇₋₁₀ aralkyl group;
R^{dN4} represents
1) hydrogen atom,
2) a C₁₋₆ alkyl group which may have a substituent,
3) an unsaturated C₂₋₁₀ alkyl group which may have a substituent,
4) a C₁₋₆ alkoxy group which may have a substituent,
5) a C₆₋₁₄ aryl group which may have a substituent,
6) a 5-membered to 14-membered heteroaryl group which may have a substituent,
7) a C₇₋₁₀ aralkyl group which may have a substituent,
8) a C₃₋₈ cycloalkyl group which may have a substituent,
9) a C₄₋₉ cycloalkylalkyl group which may have a substituent,
10) a 5-membered to 14-membered heteroaralkyl group which may have a substituent,
11) -NR^{dN6}R^{dN7} (wherein R^{dN6} and R^{dN7} are the same as or different from each other and each represents hydrogen atom or a C₁₋₆ alkyl group) or
12) a 5-membered to 14-membered non-aromatic heterocyclic group which may have a substituent; and
R^{dN5} represents
1) hydrogen atom,
2) a C₁₋₆ alkyl group which may have a substituent,
3) an unsaturated C₂₋₁₀ alkyl group which may have a substituent,
4) a C₆₋₁₄ aryl group which may have a substituent,
5) a 5 to 14-membered ring heteroaryl group which may have a substituent,
6) a C₇₋₁₀ aralkyl group which may have a substituent,
7) a C₃₋₈ cycloalkyl group which may have a substituent,
8) a C₄₋₉ cycloalkylalkyl group which may have a substituent,
9) a 5-membered to 14-membered heteroaralkyl group which may have a substituent or
10) a 5-membered to 14-membered non-aromatic heterocyclic group which may have a substituent).

7. The assay method according to claim 1, wherein R² is represented by the following formula (VIII): 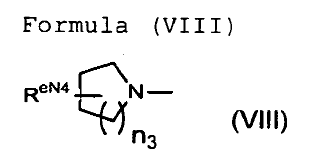 (wherein n₃ represents an integer of 1 to 3; and
R^{eN4} represents
(1) amino group,
(2) a N-C₁₋₆ alkylamino group,
(3) pyrrolidin-1-yl group,
(4) piperidin-1-yl group or
(5) morpholin-4-yl group).

8. The assay method according to claim 1, wherein R² is represented by the following formula (IX): 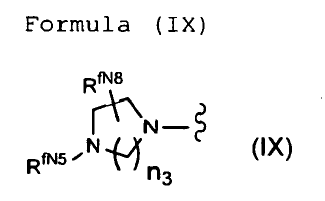 (wherein n₃ represents an integer of 1 to 3;
R^{fN8} represents
1) hydrogen atom,
2) a C₁₋₆ alkyl group,
3) a C₆₋₁₄ aryl group or
4) a C₇₋₁₀ aralkyl group; and
R^{fN5} represents
1) hydrogen atom,
2) a C₁₋₆ alkyl group which may have a substituent,
3) a C₃₋₈ cycloalkyl group which may have a substituent,
4) a 3-membered to 8-membered ring nonaromatic heterocyclic group which may have a substituent,
5) a C₆₋₁₄ aryl group which may have a substituent,
6) a 5-membered to 14-membered heteroaryl group which may have a substituent,
7) a C₇₋₁₀ aralkyl group which may have a substituent,
8) a 5-membered to 14-membered heteroaralkyl group which may have a substituent or
9) a C₄₋₉ cycloalkylalkyl group which may have a substituent).

9. The assay method according to claim 1, wherein R² is represented by the following formula (X): 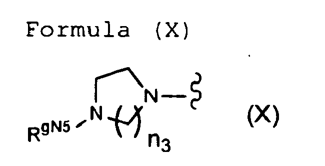 (wherein n₃ represents an integer of 1 to 3; and
R^{gN5} represents
1) hydrogen atom
2) a C₁₋₆ alkyl group which may be substituted,
3) a C₃₋₈ cycloalkyl group which may be substituted,
4) a C₄₋₉ cycloalkylalkyl group which may be substituted,
5) a C₇₋₁₀ aralkyl group which may be substituted,
6) a pyridyl group which may be substituted or
7) a tetrahydropyranyl group which may be substituted) .

10. The assay method according to claim 1, wherein the compound represented by the formula (I) is any one compound selected from the group consisting of the following compounds:
1) (8E,12E,14E)-7-acetoxy-3,6,21-trihydroxy-6,10,12,16,20-pentamethyl-18,19-epoxytricosane-8,12,14-trien-11-olide,
2) (8E, 12E, 14E)-7-((4-cycloheptylpiperazin-1-yl)-carbonyl)oxy-3,6,16,21-tetrahydroxy-6,10,12,16,20-pentamethyl-18,19-epoxytricosane-8,12,14-trien-11-olide,
3) (8E,12E,14E)-3,6,16,21-tetrahydroxy-7-((4-isopropylpiperazin-1-yl)carbonyl)oxy-6,10,12,16,20-pentamethyl-18,19-epoxytricosane-8,12,14-trien-11-olide; and
4) (8E,12E,14E)-3,6,16,21-tetrahydroxy-6,10,12,16,20-pentamethyl-7-((4-methylpiperazin-1-yl)carbonyl)oxy-18,19-epoxytricosane-8,12,14-trien-11-olide.

11. The assay method according to claim 1, comprising assaying a reduced expression of pRB, an expression of p16 or an enhanced expression of cyclin E by measuring the levels of their respective encoding mRNAs.

12. The assay method according to claim 11, wherein the method for measuring the level of the mRNAs is a quantitative RT-PCR method.

13. The assay method according to claim 11, wherein the method for measuring the level of the mRNAs is a DNA tip method.

14. The assay method according to claim 1, comprising assaying a reduced expression of pRB, an expression of p16 or an enhanced expression of cyclin E by measuring the levels of their respective proteins.

15. The assay method according to claim 14, wherein the method for measuring the levels of their respective proteins is a western blot method.

16. The assay method according to claim 14, wherein the method for measuring the levels of their respective proteins is an immunohistostaining method.

17. The assay method according to claim 14, wherein the method for measuring the levels of their respective proteins is an ELISA method

18. A kit for use in the assay method according to claim 12, comprising a compound represented by formula I and a primer that contains at least 15 consecutive base sequences of the pBR, p16 or cyclin E genes

19. A kit for use in the assay method according to claims 15, 16 or 17, comprising a compound represented by formula I and an antibody to the pRB, p16 or cyclin E.

## Patentansprüche

1. Assayverfahren zur Vorhersage der Empfindlichkeit einer Krebszelle auf eine Verbindung der folgenden Formel I, umfassend die Verwendung eines der folgenden Indizien:
1) die Expression von pRB ist vermindert;
2) p16 wird exprimiert;
3) die Expression von Cyclin E ist gesteigert;
4) die Expression von pRB ist vermindert und die Expression von Cyclin E ist gesteigert; oder
5) p16 wird exprimiert und die Expression von Cyclin E ist gesteigert: 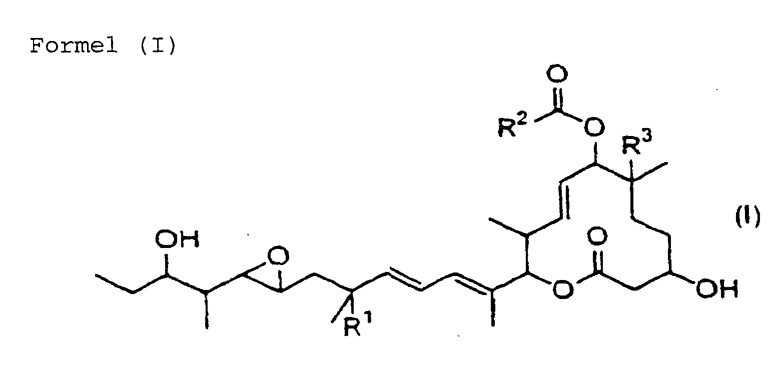
(worin R¹
(1) ein Wasserstoffatom oder
(2) eine Hydroxylgruppe darstellt;
R³
(1) ein Wasserstoffatom,
(2) eine Hydroxylgruppe oder
(3) eine C₁₋₆-Alkoxygruppe darstellt; und
R²
(1) ein Wasserstoffatom,
(2) eine C₁₋₆-Alkylgruppe, die einen Substituenten haben kann,
(3) eine C₇₋₁₀-Aralkylgruppe, die einen Substituenten haben kann,
(4) eine 5-gliedrige bis 14-gliedrige Heteroaralkylgruppe, die einen Substituenten haben Kann,
(5) die Formel (II): 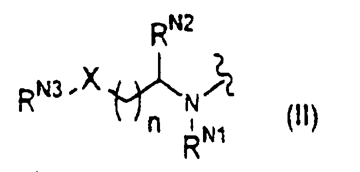 (worin
A)
n eine ganze Zahl von 0 bis 4 darstellt;
X
i) -CHR^{N4}-,
ii) -NR^{N5}- oder
iii) -O- darstellt;
R^{N1} und R^{N2} gleich oder verschieden voneinander sind und jeweils
i) ein Wasserstoffatom oder
ii) eine C₁₋₆-Alkylgruppe darstellen;
R^{N3} und R^{N4} gleich oder verschieden voneinander sind und jeweils
i) ein Wasserstoffatom,
ii) eine C₁₋₆-Alkylgruppe, die einen Substituenten haben kann,
iii) eine ungesättigte C₂₋₁₀-Alkylgruppe, die einen Substituenten haben kann,
iv) eine C₁₋₆-Alkoxygruppe, die einen Substituenten haben kann,
v) eine C₆₋₁₄-Arylgruppe, die einen Substituenten haben kann,
vi) eine 5-gliedrige bis 14-gliedrige Heteroarylgruppe, die einen Substituenten haben kann,
vii) eine C₇₋₁₀-Aralkylgruppe, die einen Substituenten haben kann,
viii) eine C₃₋₈-Cycloalkylgruppe, die einen Substituenten haben kann,
ix) eine C₄₋₉-Cycloalkylalkylgruppe, die einen Substituenten haben kann,
x) eine 5-gliedrige bis 14-gliedrige Heteroaralkylgruppe, die einen Substituenten haben kann,
xi) eine 5-gliedrige bis 14-gliedrige nicht-aromatische heterocyclische Gruppe, die einen Substituenten haben kann, oder
xii) -NR^{N6}R^{N7} darstellen (worin R^{N6} und R^{N7} gleich oder verschieden voneinander sind und jeweils ein Wasserstoffatom oder eine C₁₋₆-Alkylgruppe darstellen) oder
xiii) R^{N3} und R^{N4} über das Kohlenstoffatom, an das sie gebunden sind, miteinander verbunden sind, um eine 5-gliedrige bis 14-gliedrige nicht-aromatische heterocyclische Gruppe zu bilden, die einen Substituenten haben kann (die nicht-aromatische heterocyclische Gruppe kann einen Substituenten haben);
R^{N5}
i) ein Wasserstoffatom,
ii) eine C₁₋₆-Alkylgruppe, die einen Substituenten haben kann,
iii) eine ungesättigte C₂₋₁₀-Alkylgruppe, die einen Substituenten haben kann,
iv) eine C₆₋₁₄-Arylgruppe, die einen Substituenten haben kann,
v) eine 5-gliedrige bis 14-gliedrige Heteroarylgruppe, die einen Substituenten haben kann,
vi) eine C₇₋₁₀-Aralkylgruppe, die einen Substituenten haben kann,
vii) eine C₃₋₈-Cycloalkylgruppe, die einen Substituenten haben kann,
viii) eine C₄₋₉-Cycloalkylalkylgruppe, die einen Substituenten haben kann,
ix) eine 5-gliedrige bis 14-gliedrige Heteroaralkylgruppe, die einen Substituenten haben kann, oder
x) eine 5-gliedrige bis 14-gliedrige nicht-aromatische heterocyclische Gruppe, die einen Substituenten haben kann, darstellt oder
xi) R^{N3} und R^{N5} über das Stickstoffatom, an das sie gebunden sind, miteinander verbunden sind, um eine 5-gliedrige bis 14-gliedrige nicht-aromatische heterocyclische Gruppe zu bilden, die einen Substituenten haben kann (die nicht-aromatische heterocyclische Gruppe hat einen Substituenten),
B)
X, n, R^{N3}, R^{N4} und R^{N5} die oben definierten Gruppen darstellen;
und R^{N1} und R^{N2} eine 5-gliedrige bis 14-gliedrige nicht-aromatische heterocyclische Gruppe darstellen, worin R^{N1} und R^{N2} miteinander verbunden sind, um diese zu bilden, und die einen Substituenten haben kann,
C)
X, n, R^{N2}, R^{N4} und R^{N5} die oben definierten Gruppen darstellen;
und R^{N1} und R^{N3} eine 5-gliedrige bis 14-gliedrige nicht-aromatische heterocyclische Gruppe darstellen, worin R^{N1} und R^{N3} miteinander verbunden sind, um diese zu bilden, und die einen Substituenten haben kann, oder
D)
X, n, R^{N1}, R^{N4} und R^{N5} die oben definierten Gruppen darstellen;
und R^{N2} und R^{N3} eine 5-gliedrige bis 14-gliedrige nicht-aromatische heterocyclische Gruppe darstellen, worin R^{N2} und R^{N3} miteinander verbunden sind, um diese zu bilden, und die einen Substituenten haben kann), oder
(6) die Formel (III) darstellt: 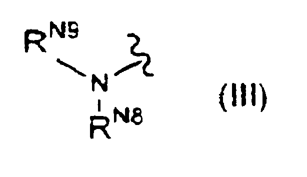
(worin R^{N8} und R^{N9} gleich oder verschieden voneinander sind und jeweils
i) ein Wasserstoffatom,
ii) eine C₁₋₆-Alkylgruppe, die einen Substituenten haben kann,
iii) eine C₆₋₁₄-Arylgruppe, die einen Substituenten haben kann,
iv) eine 5-gliedrige bis 14-gliedrige Heteroarylgruppe, die einen Substituenten haben kann,
v) eine C₇₋₁₀-Aralkylgruppe, die einen Substituenten haben kann, oder
vi) eine 5-gliedrige bis 14-gliedrige Heteroaralyklgruppe, die einen Substituenten haben kann, darstellen)).

2. Assayverfahren gemäß Anspruch 1, worin R²
1) ein Wasserstoffatom;
2) eine C₁₋₆-Alkylgruppe, die einen Substituenten haben kann,
3) eine C₇₋₁₀-Arylgruppe, die einen Substituenten haben kann, oder
4) eine 5-gliedrige bis 14-gliedrige Heteroaralkylgruppe, die einen Substituenten haben kann, ist.

3. Assayverfahren gemäß Anspruch 1, worin R² durch die folgende Formel (IV) dargestellt wird: 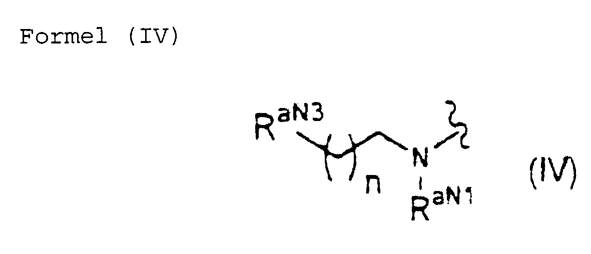 (worin n eine ganze Zahl von 0 bis 4 darstellt;
R^{aN1}
(1) ein Wasserstoff oder
(2) eine C₁₋₆-Alkylgruppe darstellt;
R^{aN3}
(1) ein Wasserstoffatom,
(2) eine N-C₁₋₆-Alkylaminogruppe,
(3) eine N,N-Di-C₁₋₆-Alkylaminogruppe,
(4) eine Ethylmethylaminogruppe,
(5) eine Pyridylgruppe,
(6) eine Pyrrolidin-1-yl-Gruppe,
(7) eine Piperidin-1-yl-Gruppe,
(8) eine Morpholin-4-yl-Gruppe oder
(9) eine 4-Methylpiperazin-1-yl-Gruppe darstellt).

4. Assayverfahren gemäß Anspruch 1, worin R² durch die folgende Formel (V) dargestellt wird: 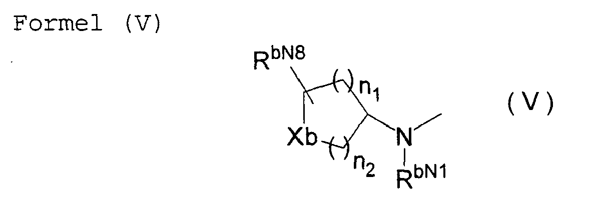 (worin n₁ und n₂ gleich oder verschieden voneinander sind und jeweils eine ganze Zahl von 0 bis 4 darstellen;
X_{b}
1) -CHR^{bN4}-,
2) -NR^{bN5}- oder
3) -O- darstellt;
R^{bN1}
1) ein Wasserstoffatom oder
2) eine C₁₋₆-Alkylgruppe darstellt;
R^{bN8}
1) ein Wasserstoffatom,
2) eine C₁₋₆-Alkylgruppe,
3) eine C₆₋₁₄-Arylgruppe oder
4) eine C₇₋₁₀-Aralkylgruppe darstellt;
R^{bN4}
1) ein Wasserstoffatom,
2) eine C₁₋₆-Alkylgruppe, die einen Substituenten haben kann,
3) eine ungesättigte C₂₋₁₀-Alkylgruppe, die einen Substituenten haben kann,
4) eine C₁₋₆-Alkyoxygruppe, die einen Substituenten haben kann,
5) eine C₆₋₁₄-Arylgruppe, die einen Substituenten haben kann,
6) eine 5-gliedrige bis 14-gliedrige Heteroarylgruppe, die einen Substituenten haben kann,
7) eine C₇₋₁₀-Aralkylgruppe, die einen Substituenten haben kann,
8) eine C₃₋₈-Cycloalkylgruppe, die einen Substituenten haben kann,
9) eine C₄₋₉-Cycloalkylalkylgruppe, die einen Substituenten haben kann,
10) eine 5-gliedrige bis 14-gliedrige Heteroaralkylgruppe, die einen Substituenten haben kann,
11) -NR^{bN6}R^{bN7} (worin R^{bN6} und R^{bN7} gleich oder verschieden voneinander sind und jeweils ein Wasserstoffatom oder eine C₁₋₆-Alkylgruppe darstellen) oder
12) eine 5-gliedrige bis 14-gliedrige nicht-aromatische heterocyclische Gruppe, die einen Substituenten haben kann, darstellt; und
R^{bN5}
1) ein Wasserstoffatom,
2) eine C₁₋₆-Alkylgruppe, die einen Substituenten haben kann,
3) eine ungesättigte C₂₋₁₀-Alkylgruppe, die einen Substituenten haben kann,
4) eine C₆₋₁₄-Arylgruppe, die einen Substituenten haben kann,
5) eine 5-gliedrige bis 14-gliedrige Heteroarylgruppe, die einen Substituenten haben kann,
6) eine C₇₋₁₀-Aralkylgruppe, die einen Substituenten haben kann,
7) eine C₃₋₈-Cycloalkylgruppe, die einen Substituenten haben kann,
8) eine C₄₋₉-Cycloalkylalkylgruppe, die einen Substituenten haben kann,
9) eine 5-gliedrige bis 14-gliedrige Heteroaralkylgruppe, die einen Substituenten haben kann, oder
10) eine 5-gliedrige bis 14-gliedrige nicht-aromatische heterocyclische Gruppe, die einen Substituenten haben kann, ist).

5. Assayverfahren gemäß Anspruch 1, worin R² dargestellt wird durch die folgende Formel (VI): 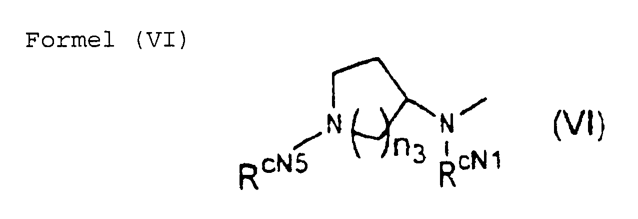 (worin n₃ eine ganze Zahl von 1 oder 2 darstellt;
R^{cN1}
(1) ein Wasserstoffatom oder
(2) eine C₁₋₆-Alkylgruppe darstellt; und
R^{cN5}
(1) ein Wasserstoffatom oder
(2) eine C₁₋₆-Alkylgruppe darstellt).

6. Assayverfahren gemäß Anspruch 1, worin R² dargestellt wird durch die folgende Formel (VII): 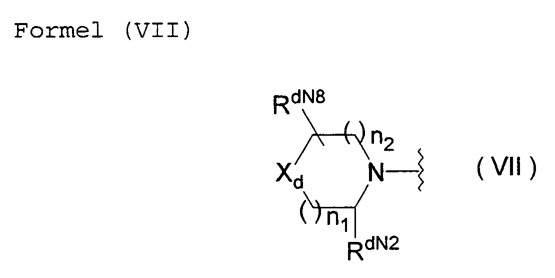 (worin n₁ und n₂ gleich oder verschieden voneinander sind und jeweils eine ganze Zahl von 0 bis 4 darstellen;
X_{d}
(1) -CHR^{dN4}-,
(2) -NR^{dN5}- oder
(3) -O- darstellt; und
R^{dN2}
(1) ein Wasserstoffatom oder
(2) eine C₁₋₆-Alkylgruppe darstellt;
R^{dN8}
(1) ein Wasserstoffatom,
(2) eine C₁₋₆-Alkylgruppe,
(3) eine C₆₋₁₄-Arylgruppe oder
(4) eine C₇₋₁₀-Aralkylgruppe darstellt;
R^{dN4}
1) ein Wasserstoffatom,
2) eine C₁₋₆-Alkylgruppe, die einen Substituenten haben kann,
3) eine ungesättigte C₂₋₁₀-Alkylgruppe, die einen Substituenten haben kann,
4) eine C₁₋₆-Alkoxygruppe, die einen Substituenten haben kann,
5) eine C₆₋₁₄-Arylgruppe, die einen Substituenten haben kann,
6) eine 5-gliedrige bis 14-gliedrige Heteroarylgruppe, die einen Substituenten haben kann,
7) eine C₇₋₁₀-Aralkylgruppe, die einen Substituenten haben kann,
8) eine C₃₋₈-Cycloalkylgruppe, die einen Substituenten haben kann,
9) eine C₄₋₉-Cycloalkylalkylgruppe, die einen Substituenten haben kann,
10) eine 5-gliedrige bis 14-gliedrige Heteroaralkylgruppe, die einen Substituenten haben kann,
11) -NR^{cN6}R^{cN7} (worin R^{cN6} und R^{cN7} gleich oder verschieden voneinander sind und jeweils ein Wasserstoffatom oder eine C₁₋₆-Alkylgruppe darstellen) oder
12) eine 5-gliedrige bis 14-gliedrige nicht-aromatische heterocyclische Gruppe, die einen Substituenten haben kann, darstellt; und
R^{dN5}
1) ein Wasserstoffatom,
2) eine C₁₋₆-Alkylgruppe, die einen Substituenten haben kann,
3) eine ungesättigte C₂₋₁₀-Alkylgruppe, die einen Substituenten haben kann,
4) eine C₆₋₁₄-Arylgruppe, die einen Substituenten haben kann,
5) eine 5- bis 14-gliedrige ringförmige Heteroarylgruppe, die einen Substituenten haben kann,
6) eine C₇₋₁₀-Aralkylgruppe, die einen Substituenten haben kann,
7) eine C₃₋₈-Cycloalkylgruppe, die einen Substituenten haben kann,
8) eine C₄₋₉-Cycloalkylalkylgruppe, die einen Substituenten haben kann,
9) eine 5-gliedrige bis 14-gliedrige Heteroaralkylgruppe, die einen Substituenten haben kann, oder
10) eine 5-gliedrige bis 14-gliedrige nicht-aromatische heterocyclische Gruppe, die einen Substituenten haben kann, darstellt).

7. Assayverfahren gemäß Anspruch 1, worin R² dargestellt wird durch die folgende Formel (VIII) 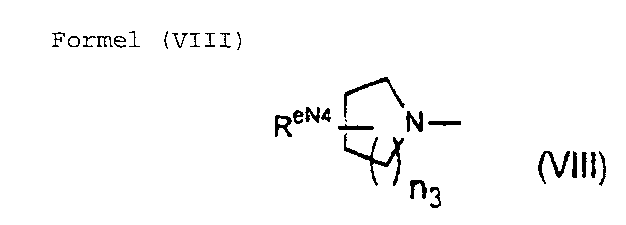 (worin n₃ eine ganze Zahl von 1 bis 3 darstellt; und
R^{eN4}
1) ein Aminogruppe,
2) eine N-C₁₋₆-Alkylaminogruppe,
3) eine Pyrrolidin-1-yl-Gruppe,
4) eine Piperidin-1-yl-Gruppe oder
5) eine Morpholin-4-yl-Gruppe darstellt).

8. Assayverfahren gemäß Anspruch 1, worin R² dargestellt wird durch die folgende Formel (IX): 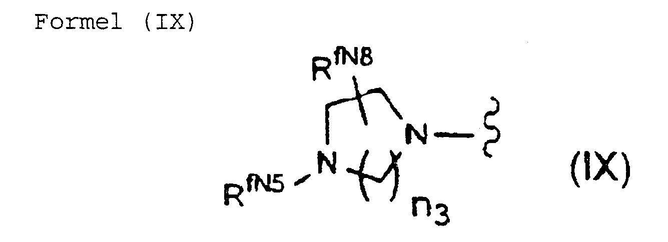 (worin n₃ eine ganze Zahl von 1 bis 3 darstellt;
R^{fN8}
1) ein Wasserstoffatom,
2) eine C₁₋₆-Alkylgruppe,
3) eine C₆₋₁₄-Arylgruppe oder
4) eine C₇₋₁₀-Aralkylgruppe darstellt; und
R^{fN5}
1) ein Wasserstoffatom,
2) eine C₁₋₆-Alkylgruppe, die einen Substituenten haben kann,
3) eine C₃₋₈-Cycloalkylgruppe, die einen Substituenten haben kann,
4) eine 3-gliedrige bis 8-gliedrige ringförmige nicht-aromatische heterocyclische Gruppe, die einen Substituenten haben kann,
5) eine C₆₋₁₄-Arylgruppe, die einen Substituenten haben kann,
6) eine 5-gliedrige bis 14-gliedrige Heteroarylgruppe, die einen Substituenten haben kann,
7) eine C₇₋₁₀-Aralkylgruppe, die einen Substituenten haben kann,
8) eine 5-gliedrige bis 14-gliedrige Heteroaralkylgruppe, die einen Substituenten haben kann oder
9) eine C₄₋₉-Cycloalkylalkylgruppe, die einen Substituenten haben kann, darstellt).

9. Assayverfahren gemäß Anspruch 1, worin R² dargestellt wird durch die folgende Formel (X) 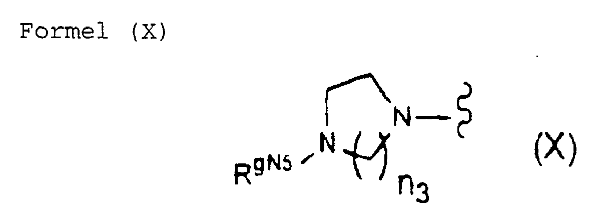 (worin n₃ eine ganze Zahl von 1 bis 3 darstellt; und
R^{gN5}
1) ein Wasserstoffatom,
2) eine C₁₋₆-Alkylgruppe, die substituiert sein kann,
3) eine C₃₋₈-Cycloalkylgruppe, die substituiert sein kann,
4) eine C₄₋₉-Cycloalkylalkylgruppe, die substituiert sein kann,
5) eine C₇₋₁₀-Aralkylgruppe, die substituiert sein kann,
6) eine Pyridylgruppe, die substituiert sein kann oder
7) eine Tetrahydropyranylgruppe, die substituiert sein kann, darstellt).

10. Assayverfahren gemäß Anspruch 1, worin die Verbindung, die durch die Formel (I) dargestellt wird, eine beliebige Verbindung, ausgewählt aus der Gruppe bestehend aus den folgenden Verbindungen, ist:
1) (8E,12E,14E)-7-Acetoxy-3,6,21-trihydroxy-6,10,12,16,20-pentamethyl-18,19-epoxytricosan-8,12,14-trien-11-olid,
2) (8E,12E,14E)-7-((4-Cycloheptylpiperazin-1-yl)-carbonyl)oxy-3,6,16,21-tetrahydroxy-6,10,12,16,20-pentamethyl-18,19-epoxytricosan-8,12,14-trien-11-olid,
3) (8E,12E,14E)-3,6,16,21-Tetrahydroxy-7-((4-isopropylpiperazin-1-yl)carbonyl)oxy-6,10,12,16,20-pentamethyl-18,19-epoxytricosan-8, 12, 14-trien-11-olid; und
4) (8E,12E,14E)-3,6,16,21-Tetrahydroxy-6,10,12,16,20-pentamethyl-7-((4-methylpiperazin-1-yl)carbonyl)oxy-18,19-epoxytricosan-8,12,14-trien-11-olid.

11. Assayverfahren gemäß Anspruch 1, umfassend das Untersuchen einer verminderten Expression von pRB, einer Expression von p16 oder einer gesteigerten Expression von Cyclin E mittels Messung der Spiegel ihrer jeweiligen kodierenden mRNAs.

12. Assayverfahren gemäß Anspruch 11, worin das Verfahren zur Messung der Spiegel der mRNAs ein quantitatives RT-PCR-Verfahren ist.

13. Assayverfahren gemäß Anspruch 11, worin das Verfahren zur Messung der Spiegel der mRNAs ein DNA-Tip-Verfahren ist.

14. Assayverfahren gemäß Anspruch 1, umfassend das Untersuchen einer verminderten Expression von pRB, einer Expression von p16 oder einer gesteigerten Expression von Cyclin E mittels Messung der Spiegel ihrer entsprechenden Proteine.

15. Assayverfahren gemäß Anspruch 14, worin das Verfahren zur Messung der Spiegel ihrer entsprechenden Proteine einen Western-Blot-Verfahren ist.

16. Assayverfahren gemäß Anspruch 14, worin das Verfahren zur Messung der Spiegel ihrer entsprechenden Proteine ein Immun-Gewebeanfärbeverfahren ist.

17. Assayverfahren gemäß Anspruch 14, worin das Verfahren zur Messung der Spiegel ihrer entsprechenden Proteine ein ELISA-Verfahren ist.

18. Kit zur Verwendung in dem Assayverfahren gemäß Anspruch 12, umfassend eine Verbindung der Formel I und einen Primer, der mindestens 15 aufeinanderfolgende Basensequenzen der pBR-, p16- oder Cyclin E-Gene enthält.

19. Kit zur Verwendung in dem Assayverfahren gemäß den Ansprüchen 15, 16 oder 17, umfassend eine Verbindung der Formel I und einen Antikörper gegen pRB, p16 oder Cyclin E.

## Revendications

1. Procédé de dosage pour prédire la sensibilité d'une cellule cancéreuse à un composé représenté par la formule I suivante, comprenant l'utilisation de n'importe quel indice parmi :
1) l'expression de pRB est réduite ;
2) p16 est exprimée ;
3) l'expression de la cycline E est augmentée ;
4) l'expression de pRB est réduite et l'expression de la cycline E est augmentée ; ou
5) p16 est exprimée et l'expression de la cycline E est augmentée : 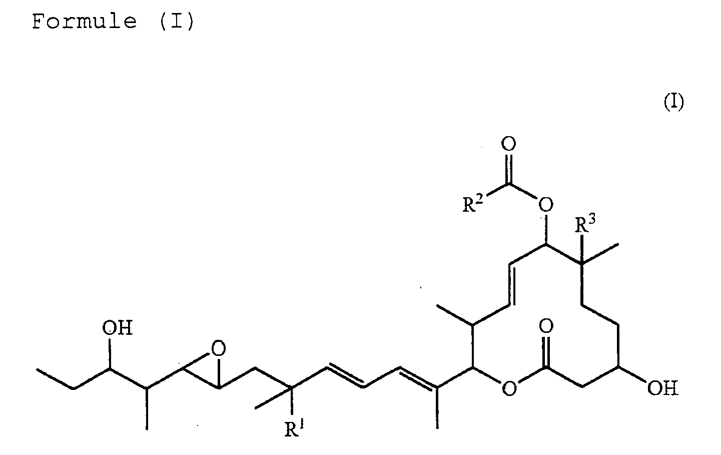
(dans laquelle, R¹ représente
(1) un atome d'hydrogène ou
(2) un groupe hydroxyle ;
R³ représente
(1) un atome d'hydrogène,
(2) un groupe hydroxyle ou
(3) un groupe alcoxy en C₁₋₆ ; et
R² représente
(1) un atome d'hydrogène,
(2) un groupe alkyle en C₁₋₆ qui peut avoir un substituant,
(3) un groupe aralkyle en C₇₋₁₀ qui peut avoir un substituant,
(4) un groupe hétéroaralkyle de 5 membres à 14 membres qui peut avoir un substituant,
(5) la formule (II) : 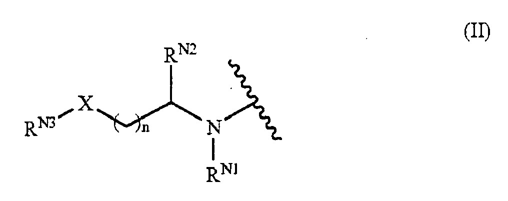 (dans laquelle,
A)
n représente un entier de 0 à 4 ;
X représente
i) -CHR^{N4}-,
ii) -NR^{N5}- ou
iii) -O- ;
R^{N1} et R^{N2} sont identiques ou différents l'un de l'autre et chacun représente
i) un atome d'hydrogène ou
ii) un groupe alkyle en C₁₋₆ ;
R^{N3} et R^{N4} sont identiques ou différents l'un de l'autre et chacun représente
i) un atome d'hydrogène,
ii) un groupe alkyle en C₁₋₆ qui peut avoir un substituant,
iii) un groupe alkyle en C₂₋₁₀ insaturé qui peut avoir un substituant,
iv) un groupe alcoxy en C₁₋₆ qui peut avoir un substituant,
v) un groupe aryle en C₆₋₁₄ qui peut avoir un substituant,
vi) un groupe hétéroaryle de 5 membres à 14 membres qui peut avoir un substituant,
vii) un groupe aralkyle en C₇₋₁₀ qui peut avoir un substituant,
viii) un groupe cycloalkyle en C₃₋₈ qui peut avoir un substituant,
ix) un groupe cycloalkylalkyle en C₄₋₉ qui peut avoir un substituant,
x) un groupe hétéroaralkyle de 5 membres à 14 membres qui peut avoir un substituant,
xi) un groupe hétérocyclique non aromatique de 5 membres à 14 membres qui peut avoir un substituant,
xii) -NRN⁶RN⁷ (dans lequel, R^{N6} et R^{N7} sont identiques ou différents l'un de l'autre et chacun représente un atome d'hydrogène ou un groupe alkyle en C₁₋₆) ou
xiii) R^{N3} et R^{N4} sont liés ensemble avec l'atome de carbone auquel ils sont liés pour former un groupe hétérocyclique non aromatique de 5 membres à 14 membres qui peut avoir un substituant (le groupe hétérocyclique non aromatique peut avoir un substituant) ;
R^{N5} représente
i) un atome d'hydrogène,
ii) un groupe alkyle en C₁₋₆ qui peut avoir un substituant,
iii) un groupe alkyle en C₂₋₁₀ insaturé qui peut avoir un substituant,
iv) un groupe aryle en C₆₋₁₄ qui peut avoir un substituant,
v) un groupe hétéroaryle de 5 membres à 14 membres qui peut avoir un substituant,
vi) un groupe aralkyle en C₇₋₁₀ qui peut avoir un substituant,
vii) un groupe cycloalkyle en C₃₋₈ qui peut avoir un substituant,
viii) un groupe cycloalkylalkyle en C₄₋₉ qui peut avoir un substituant,
ix) un groupe hétéroaralkyle de 5 membres à 14 membres qui peut avoir un substituant,
x) un groupe hétérocyclique non aromatique de 5 membres à 14 membres qui peut avoir un substituant, ou
xi) R^{N3} et R^{N5} sont liés ensemble avec l'atome d'azote auquel ils sont liés pour former un groupe hétérocyclique non aromatique de 5 membres à 14 membres qui peut avoir un substituant (le groupe hétérocyclique non aromatique peut avoir un substituant),
B)
X, n, R^{N3}, R^{N4} et R^{N5} représentent les groupes définis ci-dessus ; et R^{N1} et R^{N2} représentent un groupe hétérocyclique non aromatique de 5 membres à 14 membres auquel R^{N1} et R^{N2} sont liés ensemble pour former et qui peut avoir un substituant,
C)
X, n, R^{N2}, R^{N4} et R^{N5} représentent les groupes définis ci-dessus, et R^{N1} et R^{N3} représentent un groupe hétérocyclique non aromatique de 5 membres à 14 membres auquel R^{N1} et R^{N3} sont liés ensemble pour former et qui peut avoir un substituant, ou
D)
X, n, R^{N1}, R^{N4} et R^{N5} représentent les groupes définis ci-dessus ; et R^{N2} et R^{N3} représentent un groupe hétérocyclique non aromatique de 5 membres à 14 membres auquel R^{N2} et R^{N3} sont liés ensemble pour former et qui peut avoir un substituant), ou
(6) la formule (III) : 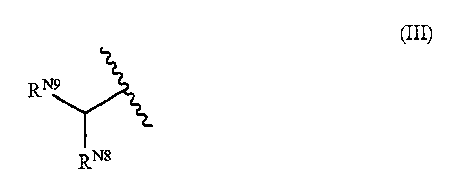 (dans laquelle, R^{N8} et R^{N9} sont identiques ou différents l'un de l'autre et chacun représente
i) un atome d'hydrogène,
ii) un groupe alkyle en C₁₋₆ qui peut avoir un substituant,
iii) un groupe aryle en C₆₋₁₄ qui peut avoir un substituant,
iv) un groupe hétéroaryle de 5 membres à 14 membres qui peut avoir un substituant
v) un groupe aralkyle en C₇₋₁₀ qui peut avoir un substituant, ou
vi) un groupe hétéroaralkyle de 5 membres à 14 membres qui peut avoir un substituant)).

2. Procédé de dosage selon la revendication 1, dans lequel R² est
1) un atome d'hydrogène ;
2) un groupe alkyle en C₁₋₆ qui peut avoir un substituant,
3) un groupe aralkyle en C₇₋₁₀ qui peut avoir un substituant ou
4) un groupe hétéroaralkyle de 5 membres à 14 membres qui peut avoir un substituant.

3. Procédé de dosage selon la revendication 1, dans lequel R² est représenté par la formule (IV) suivante : 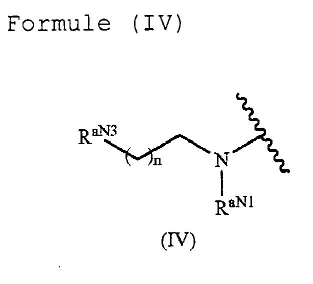 (dans laquelle n représente un entier de 0 à 4 ;
R^{aN1} représente
(1) un atome d'hydrogène ou
(2) un groupe alkyle en C₁₋₆ ;
R^{aN3} représente
(1) un atome d'hydrogène
(2) un groupe N-alkylamino en C₁₋₆,
(3) un groupe N,N-di-alkylamino en C₁₋₆,
(4) un groupe éthylméthylamino,
(5) un groupe pyridyle,
(6) un groupe pyrrolidin-1-yle,
(7) un groupe pipéridin-1-yle,
(8) un groupe morpholin-4-yle ou
(9) un groupe 4-méthylpipérazin-1-yle).

4. Procédé de dosage selon la revendication 1, dans lequel R² est représenté par la formule (V) suivante : 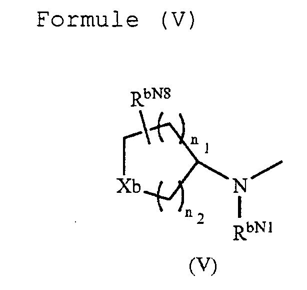 (dans laquelle n₁ et n₂ sont identiques ou différents l'un de l'autre et chacun représente un entier de 0 à 4 ;
X_{b} représente
1) -CHR^{bN4}-,
2) -NR^{bN5}- ou
3) -O- ;
R^{bN1} représente
1) un atome d'hydrogène ou
2) un groupe alkyle en C₁₋₆ ;
R^{bN8} représente
1) un atome d'hydrogène,
2) un groupe alkyle en C₁₋₆,
3) un groupe aryle en C₆₋₁₄ ou
4) un groupe aralkyle en C₇₋₁₀ ;
R^{bN4} représente
1) un atome d'hydrogène,
2) un groupe alkyle en C₁₋₆ qui peut avoir un substituant,
3) un groupe alkyle en C₂₋₁₀ insaturé qui peut avoir un substituant,
4) un groupe alcoxy en C₁₋₆ qui peut avoir un substituant,
5) un groupe aryle en C₆₋₁₄ qui peut avoir un substituant,
6) un groupe hétéroaryle de 5 membres à 14 membres qui peut avoir un substituant,
7) un groupe aralkyle en C₇₋₁₀ qui peut avoir un substituant,
8) un groupe cycloalkyle en C₃₋₈ qui peut avoir un substituant,
9) un groupe cycloalkylalkyle en C₄₋₉ qui peut avoir un substituant,
10) un groupe hétéroaralkyle de 5 membres à 14 membres qui peut avoir un substituant,
11) -NR^{bN6}R^{bN7} (dans lequel R^{bN6} et R^{bN7} sont identiques ou différents l'un de l'autre et chacun représente un atome d'hydrogène ou un groupe alkyle en C₁₋₆) ou
12) un groupe hétérocyclique non aromatique de 5 membres à 14 membres qui peut avoir un substituant ; et
R^{bN5} est
1) un atome d'hydrogène,
2) un groupe alkyle en C₁₋₆ qui peut avoir un substituant,
3) un groupe alkyle en C₂₋₁₀ insaturé qui peut avoir un substituant,
4) un groupe aryle en C₆₋₁₄ qui peut avoir un substituant,
5) un groupe hétéroaryle de 5 membres à 14 membres qui peut avoir un substituant
6) un groupe aralkyle en C₇₋₁₀ qui peut avoir un substituant,
7) un groupe cycloalkyle en C₃₋₈ qui peut avoir un substituant,
8) un groupe cycloalkylalkyle en C₄₋₉ qui peut avoir un substituant,
9) un groupe hétéroaralkyle de 5 membres à 14 membres qui peut avoir un substituant ou
10) un groupe hétérocyclique non aromatique de 5 membres à 14 membres qui peut avoir un substituant).

5. Procédé de dosage selon la revendication 1, dans lequel R² est représenté par la formule (VI) suivante : 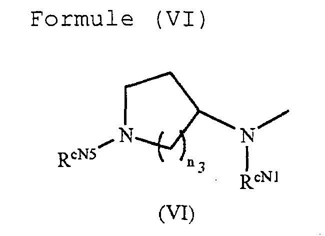 (dans laquelle n₃ représente un entier de 1 ou 2 ;
R^{cN1} représente
(1) un atome d'hydrogène ou
(2) un groupe alkyle en C₁₋₆ ;
R^{cN5} représente
(1) un atome d'hydrogène ou
(2) un groupe alkyle en C₁₋₆).

6. Procédé de dosage selon la revendication 1, dans lequel R² est représenté par la formule (VII) suivante : 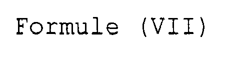 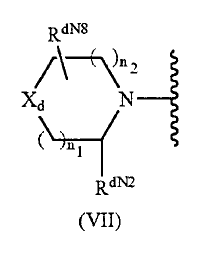 (dans laquelle n₁ et n₂ sont identiques ou différents l'un de l'autre et chacun représente un entier de 0 à 4 ;
X_{d} représente
1) -CHR^{dN}4-_{,}
2) -NR^{dN5}- ou
3) -O- ; et
R^{dN2} représente
1) un atome d'hydrogène ou
2) un groupe alkyle en C₁₋₆ ;
R^{dN8} représente
1) un atome d'hydrogène,
2) un groupe alkyle en C₁₋₆,
3) un groupe aryle en C₆₋₁₄ ou
4) un groupe aralkyle en C₇₋₁₀ ;
R^{aN4} représente
1) un atome d'hydrogène,
2) un groupe alkyle en C₁₋₆ qui peut avoir un substituant,
3) un groupe alkyle en C₂₋₁₀ insaturé qui peut avoir un substituant,
4) un groupe alcoxy en C₁₋₆ qui peut avoir un substituant,
5) un groupe aryle en C₆₋₁₄ qui peut avoir un substituant,
6) un groupe hétéroaryle de 5 membres à 14 membres qui peut avoir un substituant,
7) un groupe aralkyle en C₇₋₁₀ qui peut avoir un substituant,
8) un groupe cycloalkyle en C₃₋₈ qui peut avoir un substituant,
9) un groupe cycloalkylalkyle en C₄₋₉ qui peut avoir un substituant,
10) un groupe hétéroaralkyle de 5 membres à 14 membres qui peut avoir un substituant,
11) -NR^{dN6}R^{dN7} (dans lequel R^{dN6} et R^{dN7} sont identiques ou différents l'un de l'autre et chacun représente un atome d'hydrogène ou un groupe alkyle en C₁₋₆) ou
12) un groupe hétérocyclique non aromatique de 5 membres à 14 membres qui peut avoir un substituant ; et
R^{dN5} représente
1) un atome d'hydrogène,
2) un groupe alkyle en C₁₋₆ qui peut avoir un substituant,
3) un groupe alkyle en C₂₋₁₀ insaturé qui peut avoir un substituant,
4) un groupe aryle en C₆₋₁₄ qui peut avoir un substituant,
5) un groupe hétéroaryle à cycle de 5 à 14 membres qui peut avoir un substituant,
6) un groupe aralkyle en C₇₋₁₀ qui peut avoir un substituant,
7) un groupe cycloalkyle en C₃₋₈ qui peut avoir un substituant,
8) un groupe cycloalkylalkyle en C₄₋₉ qui peut avoir un substituant,
9) un groupe hétéroaralkyle de 5 membres à 14 membres qui peut avoir un substituant ou
10) un groupe hétérocyclique non aromatique de 5 membres à 14 membres qui peut avoir un substituant).

7. Procédé de dosage selon la revendication 1, dans lequel R² est représenté par la formule (VIII) suivante : 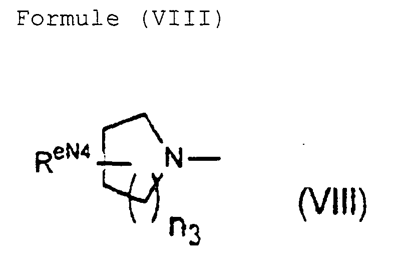 (dans laquelle n₃ un représente un entier de 1 à 3 ; et
R^{eN4} représente
(1) un groupe amino,
(2) un groupe N-alkylamino en C₁₋₆,
(3) un groupe pyrrolidin-1-yle,
(4) un groupe pipéridin-1-yle ou
(5) un groupe morpholin-4-yle).

8. Procédé de dosage selon la revendication 1, dans lequel R² est représenté par la formule (IX) suivante : 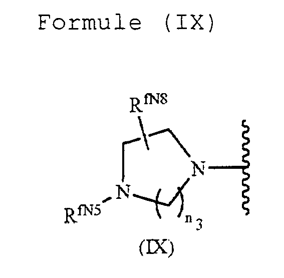 (dans laquelle n₃ représente un entier de 1 à 3 ;
R^{fN8} représente
1) un atome d'hydrogène,
2) un groupe alkyle en C₁₋₆,
3) un groupe aryle en C₆₋₁₄ ou
4) un groupe aralkyle en C₇₋₁₀ ; et
R^{fN5} représente
1) un atome d'hydrogène,
2) un groupe alkyle en C₁₋₆ qui peut avoir un substituant,
3) un groupe cycloalkyle en C₃₋₈ qui peut avoir un substituant,
4) un groupe hétérocyclique non aromatique à cycle de 3 membres à 8 membres qui peut avoir un substituant,
5) un groupe aryle en C₆₋₁₄ qui peut avoir un substituant,
6) un groupe hétéroaryle de 5 membres à 14 membres qui peut avoir un substituant,
7) un groupe aralkyle en C₇₋₁₀ qui peut avoir un substituant,
8) un groupe hétéroaralkyle de 5 membres à 14 membres qui peut avoir un substituant ou
9) un groupe cycloalkylalkyle en C₄₋₉ qui peut avoir un substituant).

9. Procédé de dosage selon la revendication 1, dans lequel R² est représenté par la formule (X) suivante : 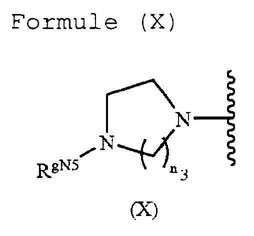 (dans laquelle n₃ représente un entier de 1 à 3 ; et
R^{gN5} représente
1) un atome d'hydrogène
2) un groupe alkyle en C₁₋₆ qui peut être substitué,
3) un groupe cycloalkyle en C₃₋₈ qui peut être substitué,
4) un groupe cycloalkylalkyle en C₄₋₉ qui peut être substitué,
5) un groupe aralkyle en C₇₋₁₀ qui peut être substitué,
6) un groupe pyridyle qui peut être substitué ou
7) un groupe tétrahydropyranyle qui peut être substitué).

10. Procédé de dosage selon la revendication 1, dans lequel le composé représenté par la formule (I) est n'importe quel composé choisi dans le groupe constitué par les composés suivants :
1) le (8E,12E,14E)-7-acétoxy-3,6,21-trihydroxy-6,10,12,16,20-pentaméthyl-18,19-époxytricosane-8,12,14-trièn-11-olide,
2) le (8E,12E,14E)-7-((4-cycloheptylpipérazin-1-yl)-carbonyl)oxy-3,6,16,21-tétrahydroxy-6,10,12,16,20-pentaméthyl-18,19-époxytricosane-8,12,14-trièn-11-olide,
3) le (8E,12E,14E)-3,6,16,21-tétrahydroxy-7-((4-isopropylpipérazin-1-yl)carbonyl)oxy-6,10,12,16,20-pentaméthyl-18,19-époxytricosane-8,12,14-trièn-11-olide;
et
4) le (8E,12E,14E)-3,6,16,21-tétrahydroxy-6,10,12,16,20-pentaméthyl-7-((4-méthylpipérazin-1-yl)carbonyl)oxy-18,19-époxytricosane-8,12,14-trièn-11-olide.

11. Procédé de dosage selon la revendication 1, comprenant le dosage d'une expression réduite de pRB, d'une expression de p16 ou d'une expression accrue de cycline E par la mesure des taux de leurs ARNm codants respectifs.

12. Procédé de dosage selon la revendication 11, dans lequel le procédé pour mesurer le taux des ARNm est un procédé par RT-PCR quantitatif.

13. Procédé de dosage selon la revendication 11, dans lequel le procédé pour mesurer le taux des ARNm est un procédé « DNA tip ».

14. Procédé de dosage selon la revendication 1, comprenant le dosage d'une expression réduite de pRB, d'une expression de p16 ou d'une expression accrue de cycline E par la mesure des taux de leurs protéines respectives.

15. Procédé de dosage selon la revendication 14, dans lequel le procédé pour mesurer les taux de leurs protéines respectives est un procédé par transfert de western.

16. Procédé de dosage selon la revendication 14, dans lequel le procédé pour mesurer les taux de leurs protéines respectives est un procédé de coloration immunohistochimique.

17. Procédé de dosage selon la revendication 14, dans lequel le procédé pour mesurer les taux de leurs protéines respectives est un procédé ELISA.

18. Kit pour une utilisation dans le procédé de dosage selon la revendication 12, comprenant un composé représenté par la formule I et une amorce qui contient au moins 15 séquences de bases consécutives des gènes de pBR, p16 ou cycline E.

19. Kit pour une utilisation dans le procédé de dosage selon la revendication 15, 16 ou 17, comprenant un composé représenté par la formule I et un anticorps contre la pRB, la p16 ou la cycline E.
